# EUROPÄISCHE PATENTSCHRIFT

(11) **EP 3 684 451 B1**
(45) Veröffentlichungstag und Bekanntmachung des Hinweises auf die Patenterteilung: **03.01.2024**
(21) Anmeldenummer: 18773130.2
(22) Anmeldetag: 14.09.2018
(51) Int. Cl.: G06F 3/04847, A61M 16/00, A61M 16/04, G06F 3/01, G06F 3/0338

(54) **VORRICHTUNG ZUR EINSTELLUNG EINES INNENDRUCKS IN EINEM ABDICHTHOHLKÖRPER**
DEVICE FOR ADJUSTING AN INTERNAL PRESSURE IN A HOLLOW SEALING BODY
DISPOSITIF DE RÉGLAGE D'UNE PRESSION INTERNE DANS UN CORPS CREUX D'ÉTANCHÉITÉ

(30) Priorität: 22.09.2017 DE 102017122045
(43) Veröffentlichungstag der Anmeldung: 29.07.2020
(73) Patentinhaber: Hamilton Medical AG, 7402 Bonaduz (CH)
(72) Erfinder: HIMMELSTOSS, Matthias, 7000 Chur (CH); LENDENMANN, Urs, 7000 Chur (CH)
(74) Vertreter: Schmitt-Nilson Schraud Waibel Wohlfrom Patentanwälte Partnerschaft mbB
(86) Internationale Anmeldenummer: PCT/EP2018/074951
(87) Internationale Veröffentlichungsnummer: WO 2019/057641

(56) Entgegenhaltungen:
- US-A1- 2010 095 961
- US-A1- 2012 268 285
- US-A1- 2017 209 664

## Beschreibung

Die vorliegende Offenbarung betrifft eine Vorrichtung zur Einstellung eines Innendrucks in einem Abdichthohlkörper, der einem distalen Abschnitt eines Trachealtubus zur Abgabe von Beatmungsluft in eine Luftröhre (Trachea) eines Patienten zugeordnet ist. Die Vorrichtung umfasst eine Bedienvorrichtung mit einem Manipulationsglied, welches zur manuellen Einstellung des Innendrucks in dem Abdichthohlkörper durch eine Bedienperson manuell betätigbar ist. Dabei wird vorgeschlagen, dass der Bedienvorrichtung eine erste und/oder eine zweite Haptik-Rückkopplungseinheit zugeordnet ist, die dazu ausgebildet ist, das Manipulationsglied im Sinne einer ersten haptischen Rückkopplung und/oder im Sinne einer zweiten haptischen Rückkopplung in Reaktion auf eine Betätigung des Manipulationsglieds zur Einstellung des Innendrucks in dem Abdichthohlkörper anzusteuern.

Die erfindungsgemäße Vorrichtung umfasst einen Trachealtubus.

Der Trachealtubus weist einen distalen Abschnitt auf, der zur Abgabe von Beatmungsluft in die Trachea ausgebildet ist, wobei der Abdichthohlkörper mit einstellbarem Innendruck dem distalen Abschnitt des Trachealtubus zugeordnet ist.

Bei der Intubierung der Trachea von Patienten mittels Trachealtubus, wie sie insbesondere bei der maschinellen Beatmung vorgenommen wird, kommt es darauf an, den Trachealtubus gegenüber der Innenwand der Trachea möglichst gut abzudichten, denn Durchsickern von Sekreten aus dem Mundraum am Trachealtubus vorbei kann zur Entwicklung von Pneumonie führen, etwa im Zuge einer länger andauernden maschinellen Beatmung. Zu diesem Zweck ist dem Trachealtubus in der Regel ein Abdichthohlkörper zugeordnet. Der Innendruck in dem Abdichthohlkörper ist von außen einstellbar. Je höher der Innendruck eingestellt ist, desto stärker wird sich der Abdichthohlkörper an die Innenwände der Trachea anlegen. Allerdings steigt mit zunehmenden Innendruck in dem Abdichthohlkörper die Gefahr von Verletzungen der Trachea. Deshalb ist man bestrebt, den niedrigsten Innendruck einzustellen, bei dem sich noch eine vernünftige Abdichtwirkung ergibt.

Bestehende Lösungen für das Einstellen des Innendrucks in dem Abdichthohlraum erfordern eine manuelle Überwachung und Anpassung des Innendrucks. Dies ist aufwändig, weil in vielen Fällen der Innendruck in recht kurzen Zeitabständen angepasst werden muss, um empfohlene Bereiche für den Innendruck einzuhalten, ohne die erforderliche Dichtigkeit zu gefährden.

Die US 2010/0078030 A1 beschreibt eine Beatmungsvorrichtung, die dazu ausgebildet ist, den Innendruck in einem um ein distales Ende eines Trachealtubus angeordneten Abdichthohlkörpers vollständig automatisch einzustellen, so dass eine manuelle Einstellung nicht mehr erforderlich ist. Der Abdichthohlkörper soll dabei mit einem genügend großen Innendruck beaufschlagt werden, um sich an die Innenwände der Trachea abdichtend anzulegen. Dabei wird der Kohlendioxidgehalt in einem Bereich der Trachea oberhalb des Abdichthohlkörpers durch einen Sensor erfasst und der Innendruck in dem Abdichthohlkörper in Reaktion auf eine Erhöhung des erfassten Kohlendioxidgehalts automatisch erhöht.

Die US 2010/0095961 A1 beschreibt eine manuelle Eingabe-Ausgabe-Vorrichtung in einem Atemgerät, das eine manuelle Eingabe zum Einstellen mindestens eines patientenkritischen Betriebsparameters des Atemgeräts hat. Die manuelle Eingabe-Ausgabe-Vorrichtung ist für eine haptische Rückmeldung programmierbar und verfügt über ein Bedienelement für die manuelle Eingabe und eine manuelle Ausgabe. Die manuelle Eingabe-Ausgabe-Vorrichtung kann zum Einstellen eines Öffnungsdruckniveaus eines einstellbaren Druckbegrenzungsventils verwendet werden.

Die US 2017/0209664 A1 beschreibt eine Atemunterstützungseinheit zum Bereitstellen von unter Druck stehender, befeuchteter Luft für einen Benutzer, welche ausgebildet ist, die Benutzerverträglichkeit zu erhöhen. Die Atemunterstützungseinheit umfasst Mittel zum Erzeugen positiver emotionaler und kognitiver Zustände eines Benutzers über die Therapie.

Die US 2012/0268285 A1 beschreibt ein System und Verfahren zum Bereitstellen von haptischem Feedback in einem medizinischen Monitor. Das haptische Feedback kann konfigurierbar sein. Beispielsweise kann haptisches Feedback nur einem definierten Satz von Ereignissen zugeordnet sein oder je nach dem Ereignis, das das Auftreten des haptischen Feedbacks auslöst, unterschiedliche Eigenschaften haben.

Die vorliegende Erfindung löst die Aufgabe, eine möglichst einfache Einstellung des Innendrucks in einem Abdichthohlkörper zum Abdichten eines Trachealtubus gegenüber einer Innenwand der Trachea Luftröhre eines Patienten zu ermöglichen, ohne die Kontrollmöglichkeiten für eine Bedienperson bei der Einstellung zu beeinträchtigen.

Zur Lösung dieser Aufgabe wird erfindungsgemäß eine Vorrichtung zur Einstellung eines Innendrucks in einem Abdichthohlkörper, der einem distalen Abschnitt eines Trachealtubus zur Abgabe von Beatmungsluft in eine Trachea eines Patienten, zugeordnet ist, vorgeschlagen. Diese Vorrichtung umfasst eine Bedienvorrichtung mit einem Manipulationsglied, welches zur manuellen Einstellung des Innendrucks in dem Abdichthohlkörper durch eine Bedienperson manuell betätigbar ist. Dabei soll der Bedienvorrichtung eine erste und/oder eine zweite Haptik-Rücckopplungseinheit zugeordnet sein, die dazu ausgebildet ist, das Manipulationsglied im Sinne einer ersten haptischen Rückkopplung und/oder im Sinne einer zweiten haptischen Rückkopplung in Reaktion auf eine Betätigung des Manipulationsglieds zur Einstellung des Innendrucks in dem Abdichthohlkörper anzusteuern.

Die vorliegende Erfindung sieht eine manuelle Einstellung des Innendrucks in dem Abdichthohlkörper durch eine Bedienperson vor, um den Trachealtubus gegenüber der Innenwand der Trachea eines Patienten einerseits ausreichend abzudichten, andererseits aber Verletzungen der Innenwand der Trachea möglichst zu vermeiden. Dabei wird durch Bereitstellen einer haptischen Rückkopplung eine möglichst intuitive Unterstützung für die Bedienperson bereitgestellt. Die erfindungsgemäße Vorrichtung bietet eine kontinuierliche Erfassung und intuitive Anpassung des Innendrucks bei Endotrachealtuben mit Abdichthohlraum sowie bei Tracheostomietuben mit Abdichthohlraum. Solche Trachealtuben werden beispielsweise während maschineller Beatmung von Erwachsenen, Kindern und Neonaten eingesetzt.

Weitere optionale Ausgestaltungen, die für alle Aspekte der vorliegenden Erfindung gelten, sollen im Folgenden näher dargelegt werden. Es versteht sich, dass jede dieser optionalen Ausgestaltungen für sich alleine zu den vorangehend ausgeführten Ausgestaltungen hinzutreten kann, dass die angesprochenen optionalen Ausgestaltungen jedoch auch in beliebiger Kombination miteinander mit den oben diskutierten Ausgestaltungen kombiniert werden können.

Die erfindungsgemäße Vorrichtung umfasst einen Trachealtubus mit einem distalen Abschnitt, der zur Abgabe von Beatmungsluft in eine Trachea eines Patienten ausgebildet ist, wobei der Abdichthohlkörper mit einstellbarem Innendruck dem distalen Abschnitt des Trachealtubus zugeordnet ist. Der Trachealtubus kann als Endotrachealtubus zum Einführen in die Luftröhre über die Rachenhöhle des Patienten oder als Tracheostomietubus zum Einführen in die Trachea mittels Luftröhrenschnitt ausgebildet sein. Der Abdichthohlkörper kann insbesondere zur Abdichtung eines Zwischenraums zwischen dem in die Trachea eingeführten distalen Abschnitt des Trachealtubus und einer Innenwand der Trachea ausgebildet sein. In bestimmten Ausführungsformen kann der Abdichthohlkörper beispielsweise die Konfiguration einer um einen Umfang am distalen Abschnitt des Trachealtubus herum angeordneten Manschette haben.

insbesondere kann in kritischen Situationen der Innendruck in dem Abdichthohlraum für einen von der Bedienperson festgelegten Zeitraum erhöht werden, um den Atemweg dicht zu halten und Aspiration bzw. eine unbeabsichtigte Extubation zu verhindern. Solche kritischen Situationen können etwa bei Erbrechen, Neuplatzierung des Trachealtubus oder Umlagerung des Patienten auftreten.

Die Vorrichtung kann beispielsweise neben dem Manipulationsglied eine Anzeigeeinheit mit Display sowie ggf. weitere Manipulationsglieder für die Bedienung aufweisen, um das Anpassen und Überprüfen der Einstellungen zu erleichtern. Vor allem soll allerdings die erfindungsgemäß vorgeschlagene haptische Rückkopplung die Bedienung für die Bedienperson intuitiv machen. Insbesondere soll die haptische Rückkopplung dabei unterstützen, die wichtigsten Parameter und/oder vorgeschlagene Zielwerte für diese Parameter permanent in schnell erfassbarer Weise anzuzeigen. Sollte eine Leckage zwischen Abdichthohlkörper und Trachea-Innenwand, oder ein anderer Fehler, beispielsweise ein zu hoher Innendruck in dem Abdichthohlkörper, festgestellt werden kann zusätzlich ein Alarm ausgelöst werden.

Die Vorrichtung kann auch dazu ausgebildet sein, auf Wunsch der Bedienperson Luft aus dem Abdichthohlkörper abzulassen, um eine sichere Extubation zu erleichtern. Darüber hinaus kann ein Absperrventil vorgesehen sein, um einen Abfall des Innendrucks in dem Abdichthohlraum im Fall einer Fehlfunktion, insbesondere im Fall einer unbeabsichtigten Trennung zwischen Abdichthohlraum Druckluftzufuhr (z.B. infolge einer undichten oder sich lösenden Verbindungsleitung) zu verhindern.

Der Innendruck in dem Abdichthohlkörper kann mittels wenigstens eines geeigneten Sensors erfasst werden. Zur Bereitstellung von Redundanz können sogar mehrere Sensoren vorgesehen sein, insbesondere zwei voneinander unabhängigen Sensoren. Der Sensor bzw. die Sensoren können insbesondere kontinuierlich erfassen, ob der eingestellte Innendruck eingehalten wird.

Es können noch weitere Sensoren vorgesehen sein, insbesondere Sensoren zur Überwachung der Dichtigkeit des Abdichthohlkörpers gegenüber der Trachea-Innenwand. Hierfür kann beispielsweise ein CO2-Sensor vorgesehen, der so angeordnet ist, dass er die CO2 Konzentration in einer Umgebung außerhalb des von dem Abdichthohlkörper abgedichteten Volumens erfasst. Ein solcher Sensor kann zum Beispiel zwischen der Rachenhöhle und dem Abdichthohlkörper angeordnet sein. Ein Anstieg des von dem Sensor erfassten CO2 Signals deutet auf Austreten von ausgeatmeter Luft und damit auf Undichtigkeit des Abdichthohlkörpers gegenüber der Trachea-Innenwand hin.

Ferner umfasst die erfindungsgemäße Vorrichtung eine Steuereinheit. Die erste Haptik-Rücckopplungseinheit und/oder die zweite Haptik-Rückkopplungseinheit können in die Steuereinheit integriert sein oder jedenfalls der Steuereinheit zugeordnet sein. Die Steuereinheit ist insbesondere so ausgebildet, dass sie Signale von Sensoren empfängt, die jeweils der ersten und/oder zweiten Haptik-Rückkopplungseinheit zugeordnet sind, und auf Grundlage dieser Signale der ersten bzw. zweiten Haptik-Rückkopplung entsprechende Signale an jeweils zur ersten Haptik-Rücckopplungseinheit oder zur zweiten Haptik-Rückkopplungseinheit gehörende Antriebe ausgibt. Diese Antriebe können in die Steuereinheit integriert sein oder aber der Steuereinheit zugeordnete eigenständige Bauteile sein. Geeignet zur Erzeugung der gewünschten ersten und/oder zweiten Haptik-Rückkopplung sind etwa Servomotore. Das Manipulationsglied zur manuellen Einstellung des Innendrucks in dem Abdichthohlkörper kann an der Steuereinheit gelagert sein.

Insbesondere kann die Steuereinheit, ggf. samt der vorangehend beschriebenen Sensorik und/oder Antriebe als portables Gerät ausgebildet sein. Auch in diesem Fall kann das Manipulationsglied an der Steuereinheit gelagert sein, so dass sich eine kompakte Einheit ergibt, die - bis auf Leitungen und Anschlüsse - praktisch alle wichtigen Teile zu Ansteuerung und Einstellung des gewünschten Innendrucks in dem Abdichthohlkörper enthält. Somit ergibt sich eine leicht transportierbare Vorrichtung zur Überwachung und Einstellung des Innendrucks in dem Abdichthohlkörper. Im Hinblick darauf, dass in vielen Fällen eine häufige Neueinstellung des Innendrucks im Abdichthohlkörper gerade in Situationen erwünscht ist, in denen ein Patient umgebettet oder transportiert wird (insbesondere per Flug, etwa im Hubschrauber) ist eine solche portable Ausgestaltung der Vorrichtung zum Einstellen des Innendrucks im Abdichthohlkörper besonders vorteilhaft. Ein solche portable Vorrichtung wird in der Regel auch so ausgebildet sein, dass sie für sich selbst betriebsfähig ist. Somit ist zum Betrieb der Vorrichtung nicht unbedingt eine Ankopplung an eine andere Maschine, etwa ein Beatmungsgerät erforderlich, auch wenn das selbstverständlich möglich ist und in vielen Fällen sinnvoll ist. Auch für eine als standortgebundenes Gerät ausgebildete Steuereinheit gilt, dass es sinnvoll sein kann, wenn die Steuereinheit für sich betriebsfähig ist. Falls gewünscht, kann eine solche Steuereinheit mit einer Beatmungsvorrichtung gekoppelt werden, so dass die Einstellung des Innendrucks im Abdichthohlkörper unter Berücksichtigung der jeweils aktuellen Beatmungsparameter und/oder Beatmungsmodi erfolgen können. Beispielsweise kann der Druck im Trachealtubus zur Einstellung des Innendrucks im Abdichthohlkörper berücksichtigt werden.

Die Steuereinheit ist erfindungsgemäß dazu ausgebildet, auf Grundlage von erfassten Werten von Kenngrößen einen Sollwert für den Innendruck in dem Abdichthohlkörper automatisiert zu bestimmen. Eine solcherart ausgebildete Steuereinheit ist der ersten Haptik-Rückkopplungseinheit zugeordnet sein, so dass die erste Haptik-Rückkopplungseinheit dazu ausgebildet ist, das Manipulationsglied im Sinne einer ersten haptischen Rückkopplung derart anzusteuern, dass eine dem von der Steuereinheit bestimmten Sollwert des Innendrucks in dem Abdichthohlkörper entsprechende Stellung des Manipulationsglieds für die Bedienperson wahrnehmbar hervorgehoben wird.

Insbesondere kann die Steuereinheit derart ausgebildet sein, dass sie wenigstens eine automatisch arbeitende Steuer/Regelprozedur zur automatischen Einstellung des Innendrucks in dem Abdichthohlkörper abarbeitet. Bei Beatmung von Patienten mittels einer Beatmungsvorrichtung kann diese automatisch arbeitende Steuer/Regelprozedur in eine automatisch arbeitende Beatmungssteuerung eingebunden sein, welche - in der Regel auf Grundlage eines bestimmten Beatmungsmodells - wenigstens eine automatisch arbeitende Steuer/Regelprozedur zur vollautomatischen Beatmung von Patienten abarbeitet. Beispiele solcher teilweise oder weitgehend vollständig automatisch arbeitender Beatmungsprozeduren sind beispielsweise unter den Namen "ASV" und "INTELLIVENT-ASV" bei Beatmungsvorrichtungen der Anmelderin bekannt. Hierzu kann die Steuereinheit in die Beatmungsvorrichtung integriert sein. Alternativ kann die Steuereinheit als an sich separates Bauteil ausgebildet sein, aber über Schnittstellen verfügen, die ein Zusammenwirken zwischen Steuereinheit und Beatmungsvorrichtung erlauben. Die automatische Beatmungsprozedur, und mit ihr auch die Steuerung/Regelung des Innendrucks in dem Abdichthohlkörper, arbeitet dabei aber gewissermaßen unsichtbar oder "im Hintergrund". Damit soll zum Ausdruck gebracht werden, dass einer oder mehrere der von der Beatmungsprozedur bzw. der Steuer/Regelprozedur zur automatischen Einstellung des Innendrucks in dem Abdichthohlkörper festgelegten Beatmungsparameter, insbesondere der Innendruck in dem Abdichthohlkörper, nicht unmittelbar durch die Beatmungsvorrichtung oder die Steuereinheit zur Beatmung bzw. zur Einstellung des Innendrucks in dem Abdichthohlkörper angewendet werden. Vielmehr sollen diese Beatmungsparameter, insbesondere der Innendruck in dem Abdichthohlkörper, weiterhin nur manuell einstellbar sein durch manuelle Betätigung des Manipulationsglieds seitens einer Bedienperson. Dennoch sollen die automatisch berechneten Beatmungsparameter, insbesondere der Innendruck in dem Abdichthohlkörper, auch wenn sie nicht unmittelbar zur automatischen Beatmung bzw. zur Einstellung des Innendrucks in dem Abdichthohlkörper angewendet werden sollen, Berücksichtigung finden, und zwar in Sinne einer haptischen Rückkopplung, die auf das Manipulationsglied im Zuge der manuellen Einstellung zurückwirkt.

Damit kann bei manueller Manipulation des Manipulationsglieds eine intuitive Unterstützung bereitgestellt werden, ohne dadurch die Kontrollmöglichkeiten für die Bedienperson zu beeinträchtigen. Dies lässt weiterhin für eine Bedienperson alle möglicherweise gewünschten manuellen Kontrollmöglichkeiten zu. Insbesondere kann eine haptische Rückmeldung auf Grundlage eines von der Beatmungsvorrichtung vorgegebenen Beatmungsparameters erfolgen, oder auf Grundlage eines durch die Beatmungsvorrichtung angewendeten Modells des Patientenzustands und/oder der jeweils einzusetzenden Beatmungsstrategie bzw. des jeweils zu wählenden Beatmungsmodus, aus dem die jeweils optimalen Beatmungsparameter bei dem jeweiligen Patientenzustand ableitbar sind. Das unterstützt das Bedienpersonal darin, teilweise sehr komplexe Zusammenhänge zwischen einzelnen Beatmungsparametern bei manueller Einstellung oder Veränderung eines Beatmungsparameters zu berücksichtigen, denn die hier vorgeschlagene Lösung verbessert die Wahrnehmbarkeit solcher Zusammenhänge, und zwar auf eine sehr intuitive Weise. Insbesondere erlaubt es die hier vorgeschlagene Lösung, solche Zusammenhänge zu erfühlen, insbesondere unmittelbar in Reaktion auf eine manuelle Manipulation des Manipulationsglieds zum Zwecke der Neueinstellung oder Nachjustierung eines der Beatmungsparameter, insbesondere des Innendrucks in dem Abdichthohlkörper. Diese Hilfestellung erhöht letztendlich die Sicherheit bei Einstellung des Innendrucks in dem Abdichthohlkörper, beispielsweise im Zusammenhang mit künstlicher Beatmung, weil das Bedienpersonal davor gewarnt wird, ungewöhnliche Einstellungen von Beatmungsparametern vorzunehmen.

Unter dem Begriff Sollwert soll sowohl ein einzelner Wert verstanden werden, als auch ein bestimmter Wertebereich oder eine Wertegrenze, etwa eine Obergrenze oder eine Untergrenze, verstanden werden. Mit dem Begriff "wahrnehmbar hervorgehoben" soll zum Ausdruck gebracht werden, dass die Bedienperson im Zuge eines Manipulationsvorgangs des Manipulationsglieds eine von der Beatmungsvorrichtung erzeugte Rückkopplung erfährt, die mittels einer beliebigen Sinneswahrnehmung erkennbar ist. Insbesondere kann die Rückkopplung fühlbar sein, also bei Kontakt mit der Beatmungseinrichtung, insbesondere bei Kontakt mit dem Manipulationsglied, eine spürbare Sinneswahrnehmung auslösen. Man spricht bei Rückkopplungen dieser Art allgemein von haptischer Rückkopplung. Insbesondere soll die haptische Rückkopplung mit demselben Organ erfassbar sein, dass zur Einstellung des Bedienparameters auf das Manipulationsglied einwirkt. Beispielsweise kann im Falle einer manuellen Einstellung des Innendrucks in dem Abdichthohlkörper mittels einer Hand des Benutzers eine haptische Rücckopplung auf die Hand, oder jedenfalls ein der Hand zugeordnetes Organ, insbesondere auf einen oder mehrere Finger oder den Handteller, wirken. Die haptische Rückkopplung kann beispielsweise im Sinne eines Widerstandes wirken, den das Manipulationsglied einer weiteren Verstellung in der vom Bediener ausgeübten Richtung entgegensetzt. Der Widerstand, den das Manipulationsglied einer Manipulation des Manipulationsglieds entgegensetzt, kann umso kleiner werden je stärker eine Manipulation des Manipulationsglieds zu einem von der Steuereinheit der Beatmungsvorrichtung bevorzugten Wert des Innendrucks in dem Abdichthohlkörper hin gerichtet ist. Das kleiner Werden kann dabei auch durchaus plötzlich oder abrupt erfolgen, um der Bedienperson das Gefühl eines "Einrastens" zu geben, wenn im Zuge eines Manipulationsvorgangs eine Stellung erreicht wird, die einem von der Steuereinheit der Beatmungsvorrichtung vorgeschlagenen Wert des Innendrucks in dem Abdichthohlkörper entspricht. Umgekehrt kann der Widerstand, den das Manipulationsglied einer Manipulation des Manipulationsglieds entgegensetzt, umso größer werden, je stärker eine Manipulation des Manipulationsglieds von einem von der Steuereinheit der Beatmungsvorrichtung bevorzugten Werts des Innendruck in dem Abdichthohlkörper weg gerichtet ist. Auch hier kann nach Überschreiten eines von der Steuereinheit vorgeschlagenen Werts des Innendrucks in dem Abdichthohlraum bei weiterer manueller Betätigung des Manipulationsglieds eine schlagartige oder abrupte Erhöhung des Widerstands erfolgen.

Die Haptik-Rückkopplungseinheit kann insbesondere einen Antrieb zum Erzeugen einer haptischen Rückkopplung aufweisen, sowie eine Sensorik zur Erfassung eines oder mehrerer Parameter, auf dessen/deren Grundlage die Haptik-Rückkopplung eingestellt wird. Die Haptik-Rückkopplungseinheit kann hierzu eine mit geeigneten Regelalgorithmen ausgebildete Steuer/Regeleinheit aufweisen. Der Antrieb kann zur Erzeugung einer geeigneten haptischen Rückkopplung, beispielsweise in Form einer Rückkopplungskraft, eines Rückkopplungsmoments, und/oder einer fühlbaren Rückkopplungsbewegung, ausgebildet sein. Die genannten Effekte können einzeln ausgeprägt sein, in vielen Fällen wird es aber günstig sein, mindestens zwei dieser Effekte zu überlagern. Eine haptische Rücckopplungskraft oder ein haptisches Rückkopplungsmoment kann beispielsweise eine Änderung eines einer Verstellung des Manipulationsglieds entgegen wirkenden Widerstands bei zunehmender oder abnehmender Verstellung des Manipulationsglieds bewirken. Eine haptische Rückkopplungsbewegung kann in Form einer verstellungsabhängigen fühlbaren Bewegung des Manipulationsglieds erfolgen, insbesondere in Form einer Vibration bei Erreichen oder in der Nähe einer Sollstellung des Manipulationsglieds. Beispielsweise eignet sich als Antrieb für die Haptik-Rückkopplungseinheit ein Servoantrieb mit geeigneter Regelung von Lage, Drehzahl und/oder Drehmoment auf Grundlage von vorbestimmten Eingangsparametern. Beispielsweise erfüllen elektronisch geregelte Servoantriebe hohe bis sehr hohen dynamische Anforderungen und sind in der Lage, eine auf das Manipulationsglied einwirkende geeignete haptische Rückkopplung unmittelbar in Reaktion auf Erfassen eines oder mehrerer Beatmungsparameter, insbesondere des Innendrucks in dem Abdichthohlkörper oder eines Anstiegs der CO2-Konzentration außerhalb des vom Abdichthohlkörper abgedichteten Volumens, und/oder auf Erfassen einer Veränderung der Stellung des Manipulationsglieds zu erzeugen. Geeignete Sensorik zur Erfassung des einen oder der mehreren Beatmungsparameter, insbesondere des Innendrucks in dem Abdichthohlkörper und/oder einer CO2-Konzentration in einem Volumen außerhalb des durch den Abdichthohlkörper abgedichteten Volumens, und/oder zur Erfassung der Stellung bzw. einer Verstellung des Manipulationsglieds aus einer Anfangsstellung wird in vielen Fällen ohnehin vorhanden sein und kann ohne Weiteres zur Erzeugung der haptischen Rückkopplung herangezogen werden. Sensorik zur Erfassung einer aktuellen Stellung des Manipulationsglieds bzw. zur Erfassung einer Verstellung der Manipulationsglieds aus einer Anfangsstellung kann beispielsweise auf Grundlage von elektrischen und/oder magnetischen Feldern, die bei Betätigung des Manipulationsglieds entstehen oder sich verändern, realisiert werden.

In diesem Zusammenhang ist darauf hinzuweisen, dass eine mit Betätigung des Manipulationsglieds einhergehende Verstellung des Manipulationsglieds aus einer Anfangsstellung nicht unbedingt sofort als geänderter Wert des Innendrucks in dem Abdichthohlkörper in der Steuerung/Regelung der von der Steuereinheit bzw. Beatmungsvorrichtung durchgeführten Beatmung des Patienten Anwendung finden muss. In vielen Fällen ist es sogar wünschenswert, dass eine mit Verstellung des Manipulationsglieds einhergehende Änderung des Innendrucks in dem Abdichthohlkörper sich solange noch nicht auswirkt, bis der jeweils neu eingestellte Parameterwert bestätigt worden ist. Diese Bestätigung kann seitens der Steuereinheit bzw. Beatmungsvorrichtung geschehen, beispielsweise nachdem die Steuereinheit bzw. Beatmungsvorrichtung erfasst hat, dass das Manipulationsglied nach Verstellung über eine vorbestimmte Zeitdauer hinweg ohne weitere Verstellung in einer neuen Stellung verblieben ist und die Steuereinheit bzw. Beatmungsvorrichtung so konfiguriert ist, dass sie diese neue Stellung dann als gewünschte Endstellung ansieht. Dann ist eine entsprechende Rückmeldung an den Anwender, dass der neu eingestellte Parameterwert von der Steuereinheit bzw. eatmungsvorrichtung übernommen worden ist, sinnvoll, etwa akustisch, visuell oder auch haptisch. Oft wird man aber zur Übernahme eines mittels Verstellen des Manipulationsglieds neu eingestellten Innendrucks in dem Abdichthohlkörper eine Bestätigung durch den Anwender selbst verlangen, etwa indem der Anwender das Manipulationsglied entsprechend betätigt oder ein weiteres Manipulationsglied betätigt. Beispielsweise kann im Falle eines als Dreh-/Drücksteller ausgebildeten Manipulationsglieds ein Verstellen des Innendrucks in dem Abdichthohlkörper durch Verdrehen des Manipulationsglieds aus einer Anfangsstellung bis zu einer dem neuen Wert entsprechenden Stellung erfolgen, während eine Bestätigung eines neu eingestellten Werts ein nachfolgendes, zusätzliches Niederdrücken des Manipulationsglieds verlangt. Erst nach dem Bestätigen wird der neu eingestellte Wert für den Innendruck in dem Abdichthohlkörper von der Beatmungsvorrichtung in der Beatmung des Patienten übernommen und fortan angewendet. Bei solcher Konfiguration ist es sinnvoll, die hier angesprochene Haptik-Rückkopplung auf Grundlage einer Verstellung des Manipulationsglieds aus einer Anfangsstellung zu erzeugen (und nicht auf Grundlage eines tatsächlich bei der Beatmung angewendeten Parameterwerts), weil der verstellte Parameterwert noch nicht von der Steuereinheit bzw. Beatmungsvorrichtung übernommen worden ist und sich die mit der Verstellung des Manipulationsglieds einhergehende Veränderung des Innendrucks in dem Abdichthohlkörper ja noch nicht auf die Beatmung auswirkt. Wenn also im Rahmen der vorliegenden Offenbarung von einem "aktuell eingestellten Wert" des Innendrucks in dem Abdichthohlkörper, oder eines anderen Beatmungsparameters, die Rede ist, so ist damit gemeint ein einer Verstellung des Manipulationsglieds entsprechender Wert des Innendrucks in dem Abdichthohlkörper, wie er sich einstellen würde, wenn der gerade eingestellte Wert bestätigt und von der Steuereinheit bzw. Beatmungsvorrichtung übernommen werden würde. Die Haptik-Rückkopplung beruht also auf einer Simulation nach Maßgabe der aktuellen Verstellung des Manipulationsglieds und gibt ggf. sogar eine solche simulierte Beatmungssituation wieder. Dies gilt gleichermaßen für die oben bereits angesprochene erste Haptik-Rückkopplung also auch für die nachfolgend noch zu erläuternde zweite Haptik-Rückkopplung.

Insbesondere kann die Steuereinheit dazu ausgebildet sein, den Sollwert für den Innendruck in dem Abdichthohlkörper fortlaufend, insbesondere synchronisiert mit jeweiligen Atemzyklen, automatisiert zu bestimmen. Damit stellt die Steuereinheit eine im Hintergrund ablaufende teilweise oder vollständig, jedenfalls weitgehend vollständig, automatisch arbeitende Prozedur bereit, die fortlaufend, insbesondere mit den Atemzyklen synchronisiert, alle den Innendruck in dem Abdichthohlkörper und ggf. auch weitere relevante Beatmungsparameter dem gerade herrschenden Patientenzustand angepasst einstellt bzw. nachstellt. Die Steuereinheit wäre im Prinzip in der Lage, den Innendruck in dem Abdichthohlkörper und ggf. auch die anderen relevanten Beatmungsparameter fortlaufend anzupassen, so dass manuelle Einstellungen bzw. Nachstellungen dieser Beatmungsparameter an sich überflüssig wären.

Wie bereits angesprochen, ist es günstig, wenn die erste Haptik-Rückkopplungseinheit dazu ausgebildet ist, das Manipulationsglied derart anzusteuern, dass die dem von der Steuereinheit bestimmten Sollwert des Innendrucks in dem Abdichthohlkörper entsprechende Stellung des Manipulationsglieds für die Bedienperson fühlbar hervorgehoben wird. Dies kann beispielsweise durch eine Rückmeldung geschehen, die der Bedienperson ein "Klick"-Gefühl oder "Einrast"-Gefühl vermittelt, wenn ein von der Steuereinheit bestimmter Sollwert erreicht oder überstrichen wird. Denkbar wäre etwa eine abrupte Veränderung eines Widerstands, den das Manipulationsglied einer Manipulationsbewegung entgegensetzt kurz vor Erreichen des Sollwerts, insbesondere eine abrupte Verringerung dieses Widerstands. Dementsprechend würde sich der Widerstand dann kurz nach Überstreichen des Sollwerts wieder abrupt in umgekehrter Richtung verändern, also insbesondere wieder abrupt ansteigen. Andere haptische Rückmeldungen sind ebenfalls denkbar, wie unten näher ausgeführt.

Erfindungsgemäß ist die erste Haptik-Rückkopplungseinheit dazu ausgebildet, eine auf Grundlage einer Abweichung zwischen dem durch manuelle Betätigung des Manipulationsglieds aktuell eingestellten Wert des Innendrucks in dem Abdichthohlkörper und dem von der Steuereinheit bestimmten Sollwert des Innendrucks in dem Abdichthohlkörper bestimmte erste haptische Rückkopplung zu erzeugen.

Wie bereits eingangs angesprochen, soll unter dem Begriff Abweichung allgemein eine wie auch immer geartete Abweichung des aktuell eingestellten Werts des Innendrucks in dem Abdichthohlkörper von dem Sollwert verstanden werden. Eine solche Abweichung kann beispielsweise als eine Differenz, ein Verhältnis oder eine andere Beziehung zwischen dem aktuell eingestellten Wert und dem Sollwert erfasst werden. Zur Umsetzung dieser Ausgestaltung kann als Abweichung eine Funktion gebildet werden, die eine die Abweichung ausdrückende Beziehung herstellt zwischen dem im Zuge eines Manipulationsvorgangs am Manipulationsglied aktuell eingestellten Wert des Innendrucks in dem Abdichthohlkörper und dem jeweils aktuell von der Steuereinheit berechneten oder abgeleiteten Sollwert für den Innendruck in dem Abdichthohlkörper. Anhand dieser Funktion kann eine haptische Rückkopplung erzeugt werden. Diese haptische Rückkopplung kann die ihr zu Grunde liegende Funktion direkt wiedergeben. Alternativ ist auch denkbar, dass dieser Funktion noch eine weitere Größe überlagert wird, beispielsweise ein abrupte Änderung der haptischen Rückkopplung bei Erreichen des Sollwerts (in der Regel wenn die Funktion einen Extremwert, insbesondere einen Minimalwert, erreicht), um ein Klick-Gefühl zu erzeugen, wie oben bereits ausführlich dargelegt.

Es sei nochmals darauf hingewiesen, dass mit dem Begriff "aktuell eingestellter Wert" des Innendrucks in dem Abdichthohlkörper ein einer gerade vorgenommenen Verstellung des Manipulationsglieds entsprechender Wert des Innendrucks in dem Abdichthohlkörper gemeint ist, wie er sich einstellen würde, wenn der gerade eingestellte Wert bestätigt und von der Beatmungsvorrichtung übernommen werden würde.

In manchen Fällen kann es günstig sein, wenn das Manipulationsglied eine Tendenz hat, von selbst eine Stellung einzunehmen, die dem durch die Steuereinheit bestimmten Sollwert des Innendrucks in dem Abdichthohlkörper entspricht. Dann kann man vorsehen, dass die erste Haptik-Rückkopplungseinheit das Manipulationsglied in einer solchen Weise beaufschlagt, dass das Manipulationsglied ohne manuelle Betätigung eine Stellung einnimmt, oder jedenfalls einzunehmen sucht, die dem durch die Steuereinheit bestimmten Sollwert des Innendrucks in dem Abdichthohlkörper entspricht. Ohne Betätigung durch eine Bedienperson, oder wenn eine solche Betätigung nicht zu Ende geführt wird, stellt das Manipulationsglied dann autonom den von der Steuereinheit vorgegeben Wert für den Innendruck in dem Abdichthohlkörper ein. Selbstverständlich kann einer solchen haptischen Rückkopplung noch eine weitere gewünschte haptische Rückkopplung - etwa das oben diskutierte Klick-Gefühl - überlagert werden. Letztendlich könnte man bei einer solchen Ausgestaltung sogar eine vollständig autonome Einstellung des Innendrucks in dem Abdichthohlkörper durch die Steuereinheit realisieren. Wichtig ist aber, dass die Bedienperson jederzeit die Möglichkeit hat, durch manuelle Betätigung einen anderen Wert für den Innendruck in dem Abdichthohlkörper einzustellen als den von der Steuereinheit vorgegebenen.

Hierbei kann unterstützend, aber auch unabhängig von den vorangehend besprochenen Varianten, auch vorgesehen sein, dass die erste Haptik-Rückkopplungseinheit das Manipulationsglied in einer solchen Weise beaufschlagt, dass das Manipulationsglied in eine Stellung zurückkehrt, oder jedenfalls in eine solche Stellung zurückzukehren sucht, die dem durch die Steuereinheit bestimmten Sollwert des Innendrucks in dem Abdichthohlkörper entspricht, wenn nach erfolgter manueller Betätigung das Manipulationsglied wieder freigegeben wird. Ein solches Verhalten des Manipulationsglieds braucht nicht notwendigerweise über den gesamten Verstellbereich des Manipulationsglieds hinweg vorgesehen zu sein. Es genügt vielmehr, wenn das Manipulationsglied sich jedenfalls in einer Umgebung um den Sollwert herum so verhält. Dann führt nämlich die die erste haptische Rückkopplung die Bedienperson zum von der Steuereinheit vorgegebenen Sollwert hin, sobald sich das Manipulationsglied im Zuge eines Manipulationsvorgangs einen vorgegebenen Bereich um den Sollwert herum erreicht. Sobald sich das Manipulationsglied in diesem Bereich befindet, zentriert sich das Manipulationsglied infolge der ersten haptischen Rückkopplung beim durch die Steuereinheit vorgegebenen Sollwert, zumindest solange keine manuelle Betätigung erfolgt.

Beispielsweise kann die erste haptische Rückkopplung das Manipulationsglied mit einer Rückstellkraft oder einem Rückstellmoment mit der Charakteristik einer um eine Ruhestellung ausgelenkten Feder, insbesondere einer um einen Ruhepunkt ausgelenkten harmonischen Feder, beaufschlagen. Diese Charakteristik kann jedenfalls in dem zuvor erwähnten Bereich um den Sollwert herum vorgesehen sein. Sie kann beispielsweise mit einer weiteren haptischen Rückkopplung kombiniert sein. Beispielsweise kann vorgesehen sein, dass die erste haptische Rückkopplung mit einer zweiten haptischen Rückkopplung kombiniert ist, die einen der gerade gewählten Stellung des Manipulationsglieds entsprechenden Wert des Innendrucks in dem Abdichthohlkörper anzeigt.

Wie bereits angesprochen kann die erste Haptik-Rückkopplungseinheit dazu ausgebildet sein, im Zuge eines manuellen Manipulationsvorgangs des Manipulationsglieds bei Überfahren einer Stellung des Manipulationsglieds, die dem von der Steuereinheit bzw. der Beatmungsvorrichtung bestimmten Sollwert des Innendrucks in dem Abdichthohlkörper entspricht, eine abrupte Änderung der ersten haptischen Rückkopplung zu erzeugen. Damit soll gemeint sein, dass eine abrupte Änderung des innendrucks in dem Abdichthohlkörper erfolgt, der die erste haptische Rückkopplung charakterisiert. Dies kann insbesondere eine Rückkopplungskraft oder ein Rückkopplungsmoment sein. Unter dem Begriff abrupt soll eine starke, insbesondere eine sprunghafte oder unstetige Veränderung des Werts für den Rückkopplungsparameter verstanden werden, beispielsweise eine sprunghafte oder unstetige Veränderung, oder jedenfalls eine zumindest näherungsweise sprunghafte oder unstetige Veränderung, einer Rückkopplungskraft oder eines Rückkopplungsmoments, die/das zur Weiterführung der manuellen Betätigung des Manipulationsglieds überwunden werden muss. Der Rückkopplungsparameter soll insbesondere bei oder kurz vor Erreichen der dem Sollwert entsprechenden Stellung des Manipulationsglieds abrupt kleiner werden und nach Überschreiten der dem Sollwert entsprechenden Stellung abrupt größer werden, so dass ein Einrastgefühl oder Klick-Gefühl bei Überstreichen des der dem Sollwert entsprechenden Stellung wahrnehmbar (insbesondere erfühlbar) ist.

In manchen Fällen kann es nützlich sein, wenn die erste Haptik-Rückkopplung davon abhängig ist, in welchem von mehreren Wertebereichen für den Innendruck in dem Abdichthohlkörper sich die Abweichung des durch manuelle Betätigung des Manipulationsglieds aktuell eingestellten Wertes des Innendrucks in dem Abdichthohlkörper bzw. des anderen Beatmungsparameters von dem von der Steuereinheit bestimmten Sollwert des Innendrucks in dem Abdichthohlkörper befindet. Man kann auf diese Weise ohne Weiteres mehrere von der Steuereinheit bevorzugte Sollwerte für den Innendruck in dem Abdichthohlkörper bzw. den anderen Beatmungsparameter haben, wobei die erste haptische Rückkopplung sich jeweils auf den Sollwert für den Innendruck in dem Abdichthohlkörper bezieht, der einem jeweiligen Bereich zugeordnet ist, in dem sich das Manipulationsglied gerade befindet. Wechselt dieser Bereich im Zuge eines Manipulationsvorgangs, so wechselt dann auch der jeweils aktuell gültige Sollwert.

Dabei können die mehreren Wertebereiche für den Innendruck in dem Abdichthohlkörper durch jeweilige Wertegrenzen voneinander getrennt sein und die Steuereinheit dazu ausgebildet sein, die Wertegrenzen zwischen den jeweiligen Wertebereichen für den Innendruck in dem Abdichthohlkörper bzw. den anderen Beatmungsparameter fortlaufend, insbesondere synchronisiert mit jeweiligen Atemzyklen, zu bestimmen. Auf diese Weise kann die jeweilige intuitive Unterstützung einer Bedienperson bei Einstellung eines oder mehrerer Beatmungsparameter automatisch an eine Veränderung des Patientenzustands angepasst werden.

Zur Erzeugung der ersten haptischen Rückkopplung, insbesondere zur Bestimmung der Abweichung zwischen dem durch manuelle Betätigung des Manipulationsglieds aktuell eingestellten Werts des Innendrucks in dem Abdichthohlkörper und dem von der Steuereinheit bestimmten Sollwert des Innendrucks in dem Abdichthohlkörper, kann der ersten Haptik-Rückkopplungseinheit wenigstens ein Sensor zugeordnet sein, der zur Erfassung der Stellung des Manipulationsglieds, insbesondere in Bezug auf eine Referenzstellung, ausgebildet ist. Ein solcher Sensor zur Erfassung der aktuellen Stellung oder Position des Manipulationsglieds wird häufig ohnehin vorgesehen sein. Dann kann die Steuereinheit anhand einer vorgegebenen Zuordnung einer aktuellen Stellung des Manipulationsglieds zu einem jeweils dieser Stellung entsprechenden Wert des Innendrucks in dem Abdichthohlkörper die Abweichung zwischen dem durch manuelle Betätigung des Manipulationsglieds aktuell eingestellten Wert des Innendrucks in dem Abdichthohlkörper und dem von der Steuereinheit bestimmten Sollwert des Innendrucks in dem Abdichthohlkörper bestimmen und die haptische Rückkopplung entsprechend einstellen.

Um der Bedienperson ein noch besseres Gefühl hinsichtlich des gerade eingestellten, oder hinsichtlich des gerade einzustellenden, Werts für den Beatmungsparameters zu vermitteln, kann man die vorangehend besprochene erste Haptik-Rückkopplung noch mit einer zweiten Haptik-Rückkopplung kombinieren, die den aktuellen Ist-Zustand des Innendrucks in dem Abdichthohlkörper anzeigt, oder jedenfalls den aktuellen Ist-Zustand des Innendrucks in dem Abdichthohlkörper bei Aktivierung des aktuell durch Manipulation des Manipulationsglieds eingestellten Werts. Hierzu kann dem Manipulationsglied eine zweite Haptik-Rückkopplungseinheit zugeordnet sein, welche das Manipulationsglied abhängig von einem Ist-Zustand des Innendrucks in dem Abdichthohlkörper im Sinne einer zweiten haptischen Rückkopplung beaufschlagt. Die erste und die zweite haptische Rückkopplung können dabei einander überlagert sein. Die erste und die zweite haptische Rückkopplung können insbesondere einen gemeinsamen Widerstand gegenüber einer (ggf. weiteren) Bewegung des Manipulationsglieds bilden. Dabei müssen die erste und die zweite Haptik-Rückopplungseinheit nicht unbedingt vollständig voneinander unabhängige Bauteile sein. Vielmehr können die erste und die zweite Haptik-Rückkopplungseinheit gemeinsame Bauteile aufweisen oder durch dieselben Bauteile gebildet sein. Insbesondere kann ein ein gemeinsamer Antrieb, der auf das Manipulationsglied wirkt, zur Erzeugung der ersten und zweiten haptischen Rückkopplung dienen.

Insbesondere kann die zweite Haptik-Rückkopplungseinheit das Manipulationsglied mit einer den Ist-Zustand des Innendrucks in dem Abdichthohlkörper anzeigenden zweiten haptischen Rückkopplung beaufschlagen. Dann ergibt sich insgesamt eine für die Bedienperson fühlbare Haptik-Rückkopplung, die einer Überlagerung aus erster Haptik-Rückkopplung und zweiter Haptik-Rückkopplung entspricht. Die Bedienperson kann somit zwei Informationen aus der Haptik-Rücckopplung entnehmen: Zum Einen, wie sich der einzustellende Innendruck in dem Abdichthohlkörper mit weiterer Verstellung des Manipulationsglieds verändern würde und zum Anderen auch, welcher Wert des Innendrucks in dem Abdichthohlkörper der in der aktuellen Situation von der Steuereinheit ausgewählte Wert wäre.

Auch der zweiten Haptik-Rückkopplungseinheit kann wenigstens ein Sensor zugeordnet sein, der zur Erfassung des Innendrucks in dem Abdichthohlkörper oder wenigstens eines anderen Beatmungsparameters ausgebildet ist. Der der zweiten Haptik-Rückkopplungseinheit zugeordnete Sensor kann auch gleichzeitig ein der ersten Haptik-Rückkopplungseinheit zugeordnetes Signal liefern, aus dem die Stellung des Manipulationsglieds in Bezug auf eine vorbestimmte Referenzstellung, insbesondere in Bezug auf die dem von der Steuereinheit bestimmten Sollwert des anderen Beatmungsparameters entsprechende Stellung des Manipulationsglieds, erfassbar ist.

Es kann hilfreich sein, Sensorik zur Erfassung weiterer Beatmungsparameter vorzusehen. Insbesondere kann es zur Einstellung eine Sollwerts für den Innendruck in dem Abdichthohlkörper sinnvoll sein, einen Sensor vorzusehen, der zur Erfassung der CO2-Konzentration in einem nicht von dem Abdichthohlkörper abgedichteten Bereich der Trachea ausgebildet ist, beispielsweise in einem Bereich der Trachea zwischen Rachen und Abdichthohlkörper. Weitere Sensoren können vorgesehen sein, beispielsweise zur Erfassung wenigstens einer der folgenden Kenngrößen: aktueller Atemwegsdruck am Atemwegseingang, aktueller Gasfluss am Atemwegseingang, kumulierter Gasfluss im Atemweg während der Inspirationsphase eines Atemzyklus, kumulierter Gasfluss im Atemweg während der Expirationsphase eines Atemzyklus, Kohlendioxidkonzentration im vom Patienten am Ende eines Atemzyklus ausgeatmeten Atemgas, arterielle Sauerstoffsättigung im Blutkreislauf des Patienten. Der Sensor kann insbesondere so ausgebildet sein, dass er die jeweilige Kenngröße kontinuierlich, oder quasi kontinuierlich erfasst, so dass sich dann aus den vom Sensor gelieferten Daten der aktuell eingestellte Wert ermitteln lässt. Selbstverständlich können zur Erfassung mehrerer Kenngrößen mehrere Sensoren vorgesehen sein, die jeweils zur Erfassung einer oder mehrerer der Kenngrößen vorgesehen sind.

Die Steuereinheit bzw. die Beatmungsvorrichtung kann insbesondere dazu ausgebildet sein, auf Grundlage der erfassten Werte von Kenngrößen einen Beatmungsmodus aus einer Mehrzahl von vorgegebenen Beatmungsmodi auszuwählen und auf Grundlage des ausgewählten Beatmungsmodus den Sollwert für den Innendruck in dem Abdichthohlkörper automatisiert zu bestimmen. Es sind eine Vielzahl aus von Beatmungsstrategien oder Beatmungsmodi bekannt, die je nach Patientenzustand angewendet werden. Es sind auch Beatmungssysteme bekannt, die automatisch, d.h. im Wesentlichen ohne manuelle Eingriffe, je nach erfasste Kenndaten, die den Zustand eines Patienten charakterisieren, zwischen verschiedenen Beatmungsmodi umschalten können. Als ein Beispiel sei auf das von der Anmelderin vertriebene Beatmungssystem "ASV" (Adapative Support Ventilation) oder "INTELLIVENT-ASV" verwiesen.

Weitere Beatmungsparameter, die im Zusammenhang mit maschineller Beatmung wichtig sind und die im Rahmen der vorliegenden Erfindung Berücksichtigung finden können, umfassen beispielsweise wenigstens einen der folgenden Parameter: Beatmungsfrequenz, Tidalvolumen, Minutenvolumen, Inspirationszeit, positiver endexpiratorischer Druck (PEEP), maximaler Atemwegsdruck, Sauerstoffkonzentration im dem Patienten zugeführten Atemgas. Man kann etwa einen Sollwert für den Innendruck in dem Abdichthohlraum abhängig von einem oder mehrerer dieser Parameter bzw. abhängig von einer Neueinstellung eines oder mehrerer dieser Parameter einstellen. Es ist auch denkbar, mehrere dieser Parameter zu kombinieren, wobei man dann in der Regel die mehren einzustellenden Parameter nacheinander mit Hilfe des hier vorgeschlagenen Systems einstellen wird.

Das Manipulationsglied kann als Einstellvorrichtung beliebiger Art ausgestaltet sein, beispielsweise als beweglicher Griff, Drehhebel, Kipphebel, Wippe oder dergleichen. Auch Eingabevorrichtungen in Form einer Tastatur, einer Maus, eines Trackballs, eines Touchpad, eines Tablets oder dergleichen sind denkbar. Häufig wird das Manipulationsglied als Drehglied ausgebildet sein oder ein als Drehglied ausgebildetes Einstellelement umfassen. Ein solches Drehglied kann insbesondere als Drehknopf ausgebildet sein, der gegen die erste und ggf. zweite Haptik-Rückkopplung manuell drehbar ist. Die erste und ggf. zweite Haptik-Rückkopplung stellt dann ein der jeweils gewünschten Rückkopplung entsprechendes Gegen-Drehmoment bereit, das zur (weiteren) Betätigung des Drehglieds überwunden werden muss. Solche Drehknöpfe sind bei vielen Beatmungsvorrichtungen zur manuellen Einstellung von Beatmungsparametern vorgesehen. Man kann in solchen Fällen im Prinzip die vorhandene Hardware des Manipulationsglieds weiter nutzen und muss nur dafür sorgen, dass das Manipulationsglied entsprechend mit der ersten Haptik-Rückkopplung und ggf. mit der zweiten Haptik-Rückkopplung beaufschlagt wird.

In bestimmten Ausführungsformen kann das Manipulationsglied als Dreh-/Drück-Steller ausgebildet sein, so dass durch unterschiedliche Art der Betätigung unterschiedliche Beatmungsparameter, insbesondere der Innendruck in dem Abdichthohlkörper eingestellt werden können. Generell kann das Manipulationsglied zur Einstellung von mehreren Beatmungsparametern ausgebildet sein. Dies kann beispielsweise realisiert werden durch Vorauswahl des jeweils gewünschten Beatmungsparameters über ein weiteres Manipulationsglied, z.B. einen Touchscreen, eine Tastatur, oder weiteres Dreh- oder Drückelement, wobei dann für den jeweils gewählten Beatmungsparameter der gewünschte Wert mit Hilfe des eigentlichen Manipulationsglieds eingestellt wird. Im Falle von Dreh-/Drück-Stellern kann die Vorauswahl eines oder mehrerer Beatmungsparameter durch entsprechende Einstellung des Manipulationsglieds selbst erfolgen, z.B. durch Einstellen des Manipulationsglieds in eine vorbestimmte Kippstellung oder Translationsstellung zur Vorauswahl des anderen Beatmungsparameters und Drehbewegung des Manipulationsglieds zur Einstellung des gewünschten Werts für den Innendruck in dem Abdichthohlkörper.

Gemäß einem weiteren Aspekt betrifft die vorliegende Erfindung auch ein Verfahren zum Bereitstellen einer unterstützenden Interaktion beim Einstellen eines Innendrucks in einem einem Trachealtubus zugeordneten Abdichthohlkörper, bei welchem ein einer Bedienvorrichtung zugeordnetes Manipulationsglied zum Einstellen des Innendrucks in dem Abdichthohlkörper durch eine Bedienperson manuell betätigt wird, und das Manipulationsglied in Reaktion auf die manuelle Betätigung des Manipulationsglieds zur Einstellung des Innendrucks dem Abdichthohlkörper im Sinne einer ersten haptischen Rückkopplung und/oder im Sinne einer nicht erfindungsgemäßen, zweiten haptischen Rückkopplung angesteuert wird.

Dieses Verfahren ist insbesondere zur Einstellung eines Innendrucks in einem Abdichthohlkörper vorgesehen, der einem distalen Abschnitt eines Trachealtubus zur Abgabe von Beatmungsgas in eine Trachea eines Patienten zugeordnet ist.

Erfindungsgemäß ist bei dem Verfahren vorgesehen, dass auf Grundlage von erfassten Werten von Kenngrößen ein Sollwert für den Innendruck in dem Abdichthohlkörper durch eine Steuereinheit automatisiert bestimmt wird, und das Manipulationsglied während der der manuellen Betätigung im Sinne der ersten haptischen Rückkopplung derart angesteuert wird, dass eine dem von der Steuereinheit bestimmten Sollwert des Innendrucks in dem Abdichthohlkörper entsprechende Stellung des Manipulationsglieds für die Bedienperson wahrnehmbar, insbesondere fühlbar, hervorgehoben wird.

Bei dem erfindungsgemäßen Verfahren ist auch vorgesehen, dass auf Grundlage von ertassten Werten von Kenngrößen ein Sollwert für den Innendruck in dem Abdichthohlkörper durch eine Steuereinheit automatisiert bestimmt wird, wobei das Manipulationsglied während der manuellen Betätigung des Manipulationsglieds mit einer auf Grundlage einer Abweichung zwischen dem durch manuelle Betätigung des Manipulationsglieds aktuell eingestellten Wert des Innendrucks in dem Abdichthohlkörper und dem von der Steuereinheit bestimmten Sollwert des Innendrucks in dem Abdichthohlkörper bestimmten ersten haptischen Rückkopplung beaufschlagt wird.

Das hier vorgeschlagene Verfahren betreffen nicht die Bedienung einer Vorrichtung zur Einstellung eines Innendrucks in dem Abdichthohlkörper an sich, sondern richtet sich vielmehr darauf, dass die Vorrichtung zur Einstellung des Innendrucks in dem Abdichthohlkörper bzw. die Beatmungsvorrichtung Informationen für Bedienpersonen auf intuitiv erfassbare Weise zu Verfügung stellt. Diese Bereitstellung von intuitiv erfassbaren Informationen erfolgt dabei automatisiert und ohne jedes Zutun seitens der Bedienperson. Insbesondere sind keinerlei Eingaben seitens der Bedienperson erforderlich. Vielmehr ist die Vorrichtung dazu ausgelegt, derartige Information im Wege einer ersten und ggf. einer nicht erfindungsgemäßen zweiten haptischen Rückkopplung automatisch und ohne weiteres Zutun seitens der Bedienperson zu erzeugen. Dies gilt für alle nachfolgend noch etwas näher erläuterten Verfahrensschritte.

Diese Verfahren können sinngemäß alle Merkmale umfassen, die vorangehend mit Bezug auf eine Vorrichtung zur Einstellung des Innendrucks in dem Abdichthohlkörper beschrieben worden sind, so dass auf die entsprechende Beschreibung verwiesen werden kann.

insbesondere kann bei den hier vorgeschlagenen Verfahren das Manipulationsglied durch die erste und ggf. eine nicht erfindungsgemäße zweite Haptik-Rückkopplungseinheit in einer solchen Weise beaufschlagt werden, dass das Manipulationsglied ohne manuelle Betätigung eine Stellung einnimmt - oder jedenfalls einzunehmen sucht -, die dem durch die Steuereinheit bestimmten Sollwert entspricht, oder dass das Manipulationsglied in eine Stellung zurückkehrt - oder jedenfalls in eine solche Stellung zurückzukehren sucht -, die dem durch die Steuereinheit bestimmten Sollwert entspricht, wenn nach erfolgter manueller Betätigung das Manipulationsglied wieder freigegeben wird. Es erfolgt dann also eine vollautomatische Einstellung des Innendrucks in dem Abdichthohlkörper entsprechend dem von der Vorrichtung vorgeschlagenen Wert.

Das Manipulationsglied kann durch die erste und ggf. zweite Haptik-Rückkopplungseinheit mit einer Rückstellkraft oder einem Rückstellmoment mit der Charakteristik einer um eine Ruhestellung ausgelenkten Feder, insbesondere einer um einen Ruhepunkt ausgelenkten harmonischen Feder, beaufschlagt werden.

Insbesondere kann bei den beschriebenen Verfahren im Zuge eines manuellen Manipulationsvorgangs des Manipulationsglieds bei Überfahren einer Stellung des Manipulationsglieds, die dem von der Steuereinheit bestimmten Sollwert des Innendrucks in dem Abdichthohlkörper entspricht, durch die erste und ggf. nicht erfindungsgemäße zweite Haptik-Rückkopplungseinheit automatisiert eine abrupte Änderung der ersten haptischen Rückkopplung zu erzeugt werden, insbesondere eine solche haptische Rückmeldung,d ein "Einrasten" oder ein "Klick-Gefühl" erzeugt.

Die erste haptische Rückkopplung kann bei dem Verfahren davon abhängig sein, in welchem von mehreren Wertebereichen für den Innendruck in dem Abdichthohlkörper tuell eingestellten Wertes von dem von der Steuereinheit bestimmten Sollwert befindet. Dabei können die mehreren Wertebereiche durch jeweilige Wertegrenzen voneinander getrennt sein und die Steuereinheit dazu ausgebildet sein, die Wertegrenzen zwischen den jeweiligen Wertebereichen fortlaufend, insbesondere synchronisiert mit jeweiligen Atemzyklen, zu bestimmen.

Wie oben beschrieben kann bei dem Verfahren das Manipulationsglied durch die nicht erfindungsgemäße zweite Haptik-Rückkopplungseinheit zusätzlich zu der durch die erste Haptik-Rückkopplungseinheit erzeugten ersten haptischen Rückkopplung abhängig von einem ist-Zustand des Innendrucks in dem Abdichthohlkörper im Sinne einer nicht erfindungsgemäßen zweiten haptischen Rückkopplung beaufschlagt werden. Dabei kann das Manipulationsglied mit einer den Ist-Zustand des Innendrucks in dem Abdichthohlkörper anzeigenden zweiten haptischen Rückkopplung beaufschlagt werden.

In einen weiteren Aspekt betrifft die vorliegende Erfindung auch ein Computerprogrammprodukt, welches Programmanweisungen enthält, bei deren Ausführung auf einer erfindungsgemäßen Vorrichtung ein Verfahren ausgeführt wird, wie es hierin beschrieben ist.

Die Erfindung wird im Folgenden anhand von Ausführungsbeispielen mit Bezug auf die Figuren näher erläutert. Dabei zeigt:
Fig. 1: Eine Ansicht einer Beatmungsvorrichtung mit integrierter Vorrichtung zur Einstellung eines Innendrucks in einem Abdichthohlkörper gemäß einer Ausführungsform.
Fig. 2: Eine Ansicht einer weiteren Beatmungsvorrichtung mit integrierter Vorrichtung zur Einstellung eines Innendrucks in einem Abdichthohlkörper gemäß einer weiteren Ausführungsform.
Fig. 3: Eine schematische Schnittansicht einer Vorrichtung zur Einstellung eines Innendrucks in einem Abdichthohlkörper gemäß einer weiteren Ausführungsform.
Fig. 3a: Ein in Fig. 3 mit "X" bezeichnetes Detail.
Fig. 4: Ein Blockdiagramm, welches wesentliche Funktionseinheiten in einer zur automatisierten Bestimmung von Beatmungsparametern ausgebildeten Steuereinheit einer Beatmungsvorrichtung gemäß Fig. 1 oder 2 darstellt, gemäß einem ersten Beatmungsmodus "ASV".
Fig. 5: Ein Blockdiagramm, welches wesentliche Funktionseinheiten in einer zur automatisierten Bestimmung von Beatmungsparametern ausgebildeten Steuereinheit einer Beatmungsvorrichtung gemäß Fig. 1 oder 2 darstellt, gemäß einem weiteren Beatmungsmodus "INTELLIVENT-ASM".
Fig. 6: In schematischer Ansicht den Verlauf eines Widerstands gegenüber einer Verstellbewegung eines Manipulationsglieds im Zuge einer manuellen Einstellung eines Innendrucks in einem Abdichthohlkörper gemäß einer Ausführungsform.
Fig. 7: In schematischer Ansicht den Verlauf eines Widerstands gegenüber einer Verstellbewegung eines Manipulationsglieds im Zuge einer manuellen Einstellung eines Innendrucks in einem Abdichthohlkörper gemäß einer weiteren Ausführungsform.

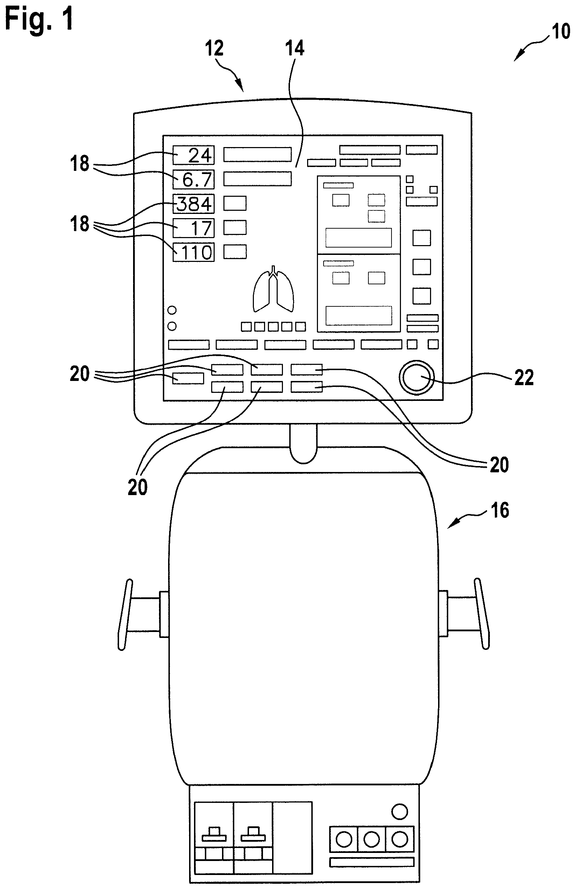

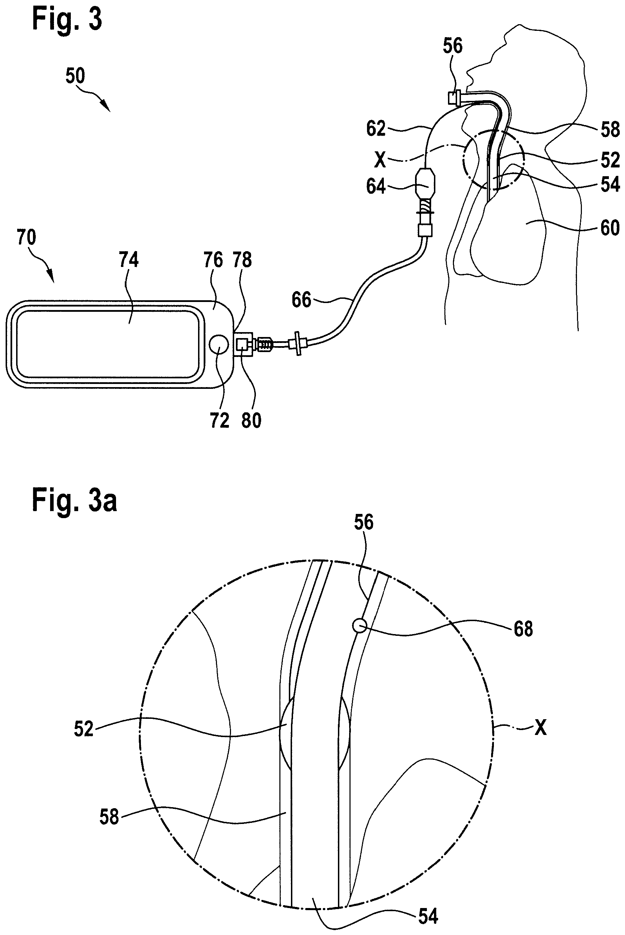

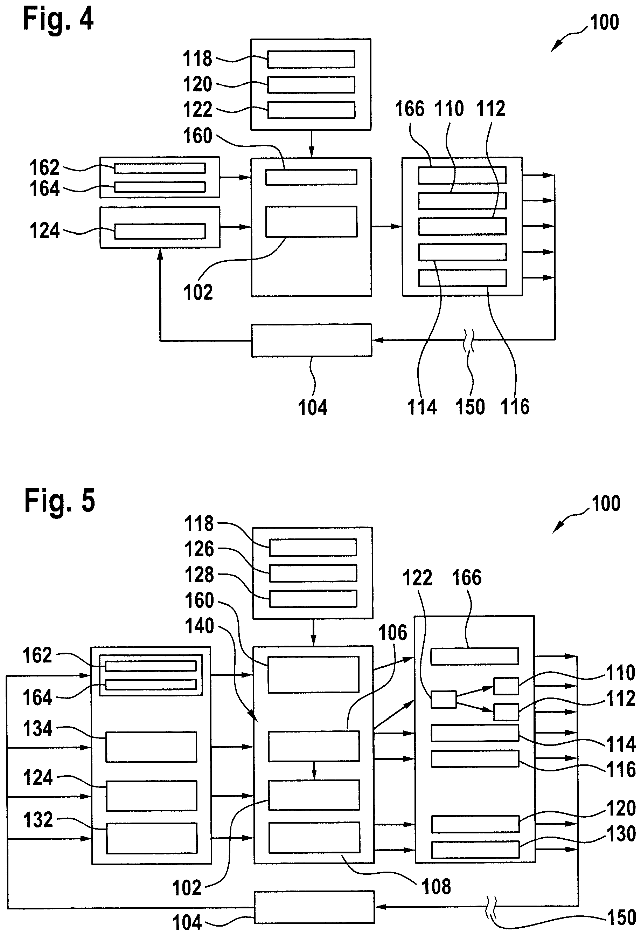

Fig. 1 zeigt eine Ansicht einer Beatmungsvorrichtung 10 gemäß einer Ausführungsform. Bei der Beatmungsvorrichtung 10 gemäß Fig. 1 ist eine Vorrichtung zur Einstellung eines Innendrucks in einem Abdichthohlkörper integriert. Die Beatmungsvorrichtung 10 weist eine Bedieneinheit 12 mit Bildschirm 14 auf, die oberhalb einer Haupteinheit 16 angeordnet ist. Die Haupteinheit 16 umfasst ein Gehäuse, in welchem die erforderlichen Vorrichtungen zur Erzeugung eines Atemgasstroms, insbesondere Pumpen, Ventile, Gasversorgungseinrichtungen und dergleichen, untergebracht sind. Die Haupteinheit 16 enthält zudem eine nicht näher dargestellte Beatmungssteuereinheit (siehe Figur 3 und 4 für eine schematische Darstellung wesentlicher Funktionsblöcke dieser Beatmungssteuereinheit), in welcher alle zur Beatmung erforderlichen Steuerprogramme und Routinen gespeichert sind und welche die jeweils zur maschinellen Beatmung erforderlichen Steuerprogramme und Routinen abarbeitet. Die Beatmungssteuereinheit enthält auch alle Routinen zur Einstellung des Innendrucks in dem Abdichthohlkörper. Der Bildschirm 14 weist eine Mehrzahl von in Fig. 1 jeweils mit 18 bezeichnete Anzeigefeldern auf, in denen vorbestimmte Beatmungsparameter dargestellt werden Zu diesen gehören für einen jeweiligen Beatmungsmodus vorgegebene Beatmungsparameter wie Beatmungsfrequenz, Tidalvolumen, Minutenvolumen, Inspirationszeit, positiver endexpiratorischer Druck (PEEP), maximaler Atemwegsdruck, Sauerstoffkonzentration im dem Patienten zugeführten Atemgas sowie insbesondere der Innendruck in dem Abdichthohlkörper. Auch Werte für bestimmte physiologische Parameter oder den Beatmungszustand kennzeichnende Parameter, die von Sensoren gemessen werden, können dort dargestellt sein wie aktueller Atemwegsdruck am Atemwegseingang, aktueller Gasfluss am Atemwegseingang, kumulierter Gasfluss im Atemweg während der Inspirationsphase eines Atemzyklus, kumulierter Gasfluss im Atemweg während der Expirationsphase eines Atemzyklus, Kohlendioxidkonzentration im vom Patienten am Ende eines Atemzyklus ausgeatmeten Atemgas, arterielle Sauerstoffsättigung im Blutkreislauf des Patienten. Von Bedeutung im Zusammenhang mit der vorliegenden Erfindung ist außerdem noch die CO2-Konzentration in einem Bereich außerhalb des durch den Abdichthohlkörper abgedichteten Volumens. Diese Konzentration kann beispielsweise durch einen zwischen Rachenhöhle und Abdichthohlkörper angeordneten CO2 Sensor erfasste werden (siehe Figur 3). Außerdem sind noch graphische Anzeigefelder vorhanden, die in Fig. 1 nicht im Einzelnen mit Bezugszeichen versehen sind. In diesen graphischen Anzeigefeldern sind weitere Informationen zur laufenden Beatmungsprozedur dargestellt, etwa eine schematische Darstellung der Lunge, aus der Informationen zu physiologischen Parametern wie Resistance R oder Compliance C wenigstens qualitativ ersichtlich sind, oder graphische Darstellungen des zeitlichen Verlaufs bestimmter sich über einen Atemzyklus hinweg charakteristisch veränderlicher Größen wie Puls, Blutdruck, Atemwegsdruck, Sauerstoffsättigung im But o.ä.

Zudem weist die Bedieneinheit 12 eine Mehrzahl von Eingabefeldern auf, die in Fig. 1 jeweils mit 20 bezeichnet sind. Über diese Eingabefelder 20 können bestimmte Befehle eingegeben werden, beispielsweise Ausdrucke der gerade am Bildschirm 14 angezeigten Funktionen, Versetzen der Beatmungsvorrichtung in Standby-Modus, Bildschirm sperren, Alarmsignal unterdrücken, Beatmung mit reinem Sauerstoff, u.ä. Funktionen. Die Eingabefelder 20 können als mechanische, elektrische oder optische Eingabevorrichtungen ausgebildet sein, wie Tasten, Knöpfe, Schalter und dergleichen. In manchen Ausführungsformen kann der Bildschirm 14 als Touchscreen ausgebildet sein, so dass Eingabe über die Eingabefelder 20 seitens einer Bedienperson durch Berühren des Bildschirms 14 eingegeben werden können. Es kann auch vorgesehen sein, dass bestimmte Eingaben durch Berühren des Bildschirms 14 innerhalb der nicht bezeichneten graphischen Anzeigefelder gemacht werden können.

Neben den genannten Bedien- und Anzeigeelementen 18, 20 weist die Bedieneinheit 12 noch einen als Manipulationsglied dienenden Hauptbedienknopf 22 auf. Dieser Hauptbedienknopf 22 dient der manuellen Einstellung von Beatmungsmodi sowie der manuellen Einstellung von Beatmungsparametern, insbesondere des Innendrucks in dem Abdichthohlkörper, durch eine Bedienperson. Der Hauptbedienknopf 22 ist damit ein Beispiel für ein Manipulationsglied, welches zur manuellen Einstellung des Innendrucks in dem Abdichthohlkörper durch eine Bedienperson manuell betätigbar ist. Der Hauptbedienknopf 22 ist in der gezeigten Ausführungsform als Dreh-/Drücksteller ausgebildet. Eine Bedienperson kann nach Auswahl eines bestimmten Beatmungsparameters, insbesondere des Innendrucks in dem Abdichthohlkörper, z.B. durch Berühren des dem Innendruck in dem Abdichthohlkörper zugeordneten Bedienfeldes 18, durch Verdrehen des Hauptbedienknopfes 22 einen jeweils eingestellten Wert für den Innendruck in dem Abdichthohlkörper verändern und damit einen neuen gewünschten Wert einstellen. Nach Abschluss des Einstellvorgangs drückt die Bedienperson auf den Hauptbedienknopf (d.h. die Bedienperson führt eine translatorische Betätigung des Hauptbedienknopfs durch), um den neu eingestellten Wert zu bestätigen. Nach erfolgter Bestätigung wird die Beatmung mit dem neu eingestellten Beatmungsparameter, beispielsweise einem höheren oder niedrigeren Wert für Innendruck in dem Abdichthohlkörper, weitergeführt.

Während des Einstellvorgangs kann die Bedienperson den sich verändernden Wert des neu einzustellenden Innendrucks in dem Abdichthohlkörper im jeweiligen Anzeigefeld 18 des Bildschirms 14 erkennen. Die Beatmungsvorrichtung 10 gemäß der hier beschriebenen Art erlaubt aber noch eine weit bequemere und intuitivere Art der Rückmeldung eines einzustellenden Innendrucks in dem Abdichthohlkörper, nämlich durch eine haptische Rückkopplung, die auf den Hauptbedienknopf 22 im Zuge einer Manipulationsbewegung zurückwirkt. In dem gezeigten Beispiel mit als Dreh-/Drücksteller ausgebildetem Hauptbedienknopf 22 wirkt die haptische Rückkopplung als ein Widerstand oder ein Gegen-Drehmoment, der durch den Hauptbedienknopf 22 gegenüber einer von einer Bedienperson erzwungenen Verdrehbewegung aus einer Ausgangsstellung A in Richtung zu einer Endstellung E ausgeübt wird.

Es versteht sich, dass eine entsprechende haptische Rückkopplung auch bei einer Umkehrung der Bewegungsrichtung auf den Hauptbedienknopf 22 ausgeübt wird. Kehrt die Bedienperson die Drehrichtung am Hauptbedienknopf 22 im Zuge eines Manipulationsvorgangs um, so kehrt sich auch die Richtung der haptischen Rückkopplung um, d.h. der nunmehr in Rückwärtsrichtung erfolgenden Betätigung des Hauptbedienknopfs (Drehung in umgekehrter Richtung) wird wieder ein entsprechender Widerstand entgegensetzt.

Der Hauptbedienknopf 22 lässt sich also nach einmal erfolgter Auswahl des Innendrucks in dem Abdichthohlkörper als neu einzustellender Beatmungsparameter nicht mehr frei drehen, sondern nur mit einer gewissen Kraft bzw. einem gewissen Drehmoment, das zur Überwindung der durch die haptische Rückkopplung ausgeübten Kraft bzw. Drehmoments erforderlich ist. Zur Erzeugung der haptischen Rückkopplung weist die Beatmungsvorrichtung 10 eine nicht im Einzelnen in den Figuren gezeigte Haptik-Rückkopplungseinheit auf, die beispielsweise einen auf eine Drehachse des Hauptbedienknopfs 22 wirkenden Servomotor und diesem zugeordnete Sensorik zur Erfassung der aktuellen Drehstellung des Hauptbedienknopfs 22 umfasst. Der Servomotor 22 kann beispielsweise durch die Steuereinheit 100 der Beatmungsvorrichtung 10 angesteuert werden, der normalerweise auch die von der Sensorik der Haptik-Rückkopplungseinheit gelieferten Werte, neben den von weiteren Sensoren der Beatmungsvorrichtung 10 gelieferten Werten, zugeführt werden.

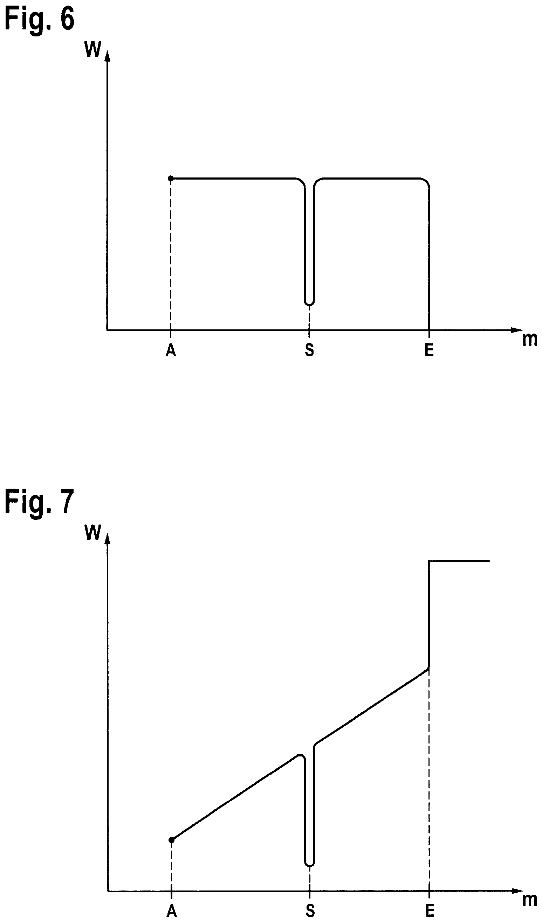

Die von der Haptik-Rückkopplungseinheit gelieferte Haptik-Rückkopplung umfasst eine erste Haptik-Rückkopplung (siehe Fig. 6), die dazu ausgebildet ist, den Hauptbedienknopf 22 im Sinne einer ersten haptischen Rückkopplung derart anzusteuern, dass eine dem von der Steuereinheit bestimmten Sollwert des Innendrucks in dem Abdichthohlkörper entsprechende Stellung des Hauptbedienknopfes 22 für die Bedienperson wahrnehmbar hervorgehoben wird. Alternativ oder zusätzlich kann die erste Haptik-Rückkopplungseinheit dazu ausgebildet sein, den Hauptbedienknopf 22 derart anzusteuern, dass die dem von der Steuereinheit bestimmten Sollwert des Innendrucks in dem Abdichthohlkörper entsprechende Stellung des Hauptbedienknopfes 22 für die Bedienperson fühlbar hervorgehoben wird.

Außerdem umfasst die Haptik-Rückkopplungseinheit noch eine zweite Haptik-Rückkopplungseinheit, die eine zweite haptische Rückmeldung liefert, die den Hauptbedienknopf 22 abhängig von einem Ist-Zustand des Innendrucks in dem Abdichthohlkörper beaufschlagt. Die zweite haptische Rückkopplung ergibt damit ein fühlbares Signal entsprechend der Verstellung des Hauptbedienknopfs 22 im Zuge einer Manipulation, das die damit einhergehende Veränderung des einzustellenden Beatmungsparameters erfühlbar werden lässt.

Insgesamt ergibt sich dann eine haptische Rückkopplung, die einer Überlagerung der ersten haptischen Rückkopplung mit der zweiten haptischen Rückkopplung entspricht, wie dies in Fig. 7 gezeigt ist und mit Bezug zu Fig. 7 nachfolgend noch näher erläutert wird. Es versteht sich, dass in weiteren Ausführungsformen die haptische Rückkopplung nur eine erste haptische Rückkopplung oder nur eine zweite haptische Rückkopplung umfassen kann.

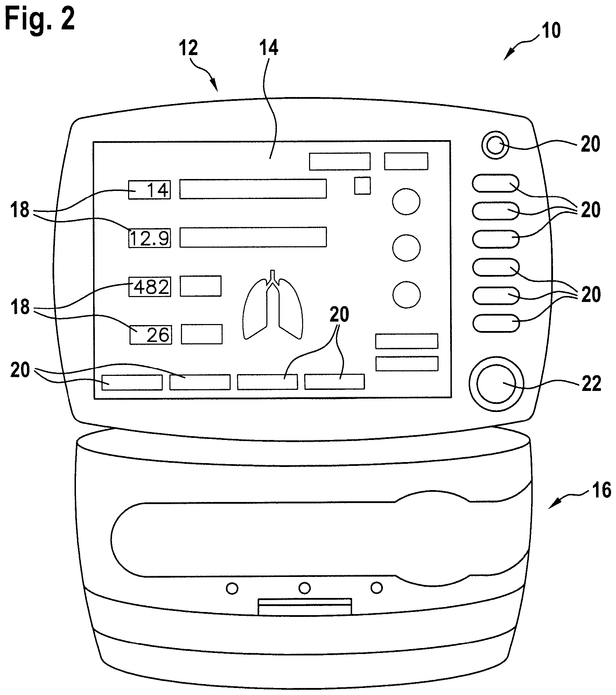

Fig. 2 zeigt eine Ansicht einer Beatmungsvorrichtung 10 gemäß einer weiteren Ausführungsform. Die Ansicht gemäß Fig. 2 entspricht im Wesentlichen Fig. 1. Gleiche Komponenten, oder jedenfalls Komponenten, die dieselbe Funktion haben wie in Fig. 1, sind mit denselben Bezugszeichen versehen. Hinsichtlich einer Beschreibung kann auf die Fig. 1 verwiesen, die insoweit ohne Weiteres auf in Bezug zu Fig. 2 gilt, es sei denn es ist ausdrücklich etwas anderes gesagt.

Fig. 3 zeigt in einer stark vereinfachten und schematischen Schnittansicht eine Vorrichtung 50 zur Einstellung eines Innendrucks in einem Abdichthohlkörper 52 gemäß einer weiteren Ausführungsform. Zum besseren Verständnis ist ein in Fig. 3 mit X bezeichnetes Detail in Fig. 3a vergrößert dargestellt.

Die Vorrichtung 50 zur Einstellung eines Innendrucks in einem Abdichthohlkörper 52 gemäß Fig. 3 ist nicht in eine Beatmungsvorrichtung integriert, sondern kann im Prinzip unabhängig von einer Beatmungsvorrichtung arbeiten. Damit kann mittels der Vorrichtung 50 der Innendruck in dem Abdichthohlkörper 52 bei einem intubierten Patienten eingestellt und überwacht werden unabhängig davon, ob oder ggf. in welchem Beatmungsmodus der Patient beatmet wird. Falls gewünscht kann die Vorrichtung 50 auch mit einer Beatmungsvorrichtung gekoppelt werden, so dass die jeweils gewählte Einstellung des Innendrucks in dem Abdichthohlkörper 52 bei der Beatmung berücksichtigt werden kann und umgekehrt der Innendruck im Abdichthohlkörper 52 an die bei der Beatmung eingestellten Verhältnisse angepasst werden kann, insbesondere an die bei der Beatmung auftretenden Druckverhältnisse im Trachealtubus 56.

Die Vorrichtung 50 weist eine Bedien/Steuereinheit 70 auf mit einem Manipulationsglied 72 und einem Bildschirm 74. Die Bedien/Steuereinheit 70 umfasst auch die nötigen Vorrichtungen zur Steuerung der Einstellung des Innendrucks in dem Abdichthohlkörper 52, insbesondere Controller (Mikroprozessor), Speicher, und die erforderliche Software. In der Bedien/Steuereinheit 70 sind insbesondere die zur Beaufschlagung des Abdichthohlkörpers 52 mit Druckluft erforderlichen Steuerprogramme und Routinen gespeichert, und die Bedien/Steuereinheit 70 ist dazu ausgebildet, die jeweils erforderlichen Steuerprogramme und Routinen abzuarbeiten. Der Bildschirm 74 weist eine Mehrzahl von Anzeigefeldern auf, in denen vorbestimmte Parameter dargestellt werden. Der Bildschirm kann zudem als Touchscreen ausgebildet sein, über den bestimmte Parameter eingegeben werden können. In der Bedien/Steuereinheit 70 sind darüber hinaus die zur Beaufschlagung des Abdichthohlkörpers 52 mit Druckluft oder Druckgas erforderlichen Vorrichtungen untergebracht, insbesondere Pumpen, Ventile, Luft/Gasversorgungseinrichtungen und dergleichen. Die Bedien/Steuereinheit 70 weist ein portables Gehäuse 76 auf, an dem ein Anschluss 78 für einen Druckluftschlauch 66 vorgesehen ist. Dem Anschluss 78 ist ein Absperrventil 80 zugeordnet. Der Druckluftschluftschlauch 66 führt über eine Druckluftleitung 62 in einen von dem Abdichthohlkörper 52 umschlossenen Raum zur Beaufschlagung desselben mit Druckluft.

Das Manipulationsglied 72 entspricht hinsichtlich seines Aufbaus und seiner Funktion im Wesentlichen dem Hauptbedienknopf 22 bei den in Fig. 1 und 2 gezeigten Beatmungsgeräten, mit der Maßgabe, dass das Manipulationsglied 72 der manuellen Einstellung des Innendrucks in dem Abdichthohlkörper 52 durch eine Bedienperson dient. Auch das Manipulationsglied 72 ist als Dreh-/Drücksteller ausgebildet. Eine Bedienperson kann durch Verdrehen des Manipulationsglieds 72 einen jeweils eingestellten Wert für den Innendruck in dem Abdichthohlkörper verändern und damit einen neuen gewünschten Wert einstellen. Die Vorrichtung 50 gemäß der hier beschriebenen Art erlaubt eine bequeme und intuitive Art der Rückmeldung eines einzustellenden Innendrucks in dem Abdichthohlkörper während des Einstellvorgangs, nämlich durch eine haptische Rückkopplung, die auf das Manipulationsglied 72 im Zuge einer Manipulationsbewegung zurückwirkt. Die Haptik-Rückkopplung kann eine erste Haptik-Rückkopplung (siehe Fig. 6) umfassen, die dazu ausgebildet ist, das Manipulationsglied 72 im Sinne einer ersten haptischen Rückkopplung derart anzusteuern, dass eine dem von der Steuereinheit 70 bestimmten Sollwert des Innendrucks in dem Abdichthohlkörper 52 entsprechende Stellung des Manipulationsglieds 72 für die Bedienperson wahrnehmbar, insbesondere fühlbar, hervorgehoben wird. Alternativ oder zusätzlich kann eine zweite haptische Rückmeldung geliefert werden, die das Manipulationsglied 72 abhängig von einem Ist-Zustand des Innendrucks in dem Abdichthohlkörper 52 beaufschlagt, insbesondere mit einem dem Ist-Zustand entsprechenden haptischen Signal. Insgesamt ergibt sich dann eine haptische Rücckopplung, die einer Überlagerung der ersten haptischen Rückkopplung mit der zweiten haptischen Rückkopplung entspricht, wie dies in Fig. 7 gezeigt ist und mit Bezug zu Fig. 7 nachfolgend noch näher erläutert wird. Es versteht sich, dass in weiteren Ausführungsformen die haptische Rückkopplung nur eine erste haptische Rückkopplung oder nur eine zweite haptische Rückkopplung umfassen kann.

In dem gezeigten Beispiel mit als Dreh-/Drücksteller ausgebildetem Manipulationsglied 72 wirkt die haptische Rückkopplung als ein Widerstand oder ein Gegen-Drehmoment, der durch das Manipulationsglied 72 gegenüber einer von einer Bedienperson erzwungenen Verdrehbewegung aus einer Ausgangsstellung A in Richtung zu einer Endstellung E ausgeübt wird. Nach Abschluss des Einstellvorgangs drückt die Bedienperson auf das Manipulationsglied 72, um den neu eingestellten Wert zu bestätigen.

Wegen weiterer Einzelheiten wird zur Vermeidung von Wiederholungen auf die Beschreibung des Hauptbedienknopfs 22 gemäß Figur 1 verwiesen, die in gleicher Weise für das Manipulationsglied 72 gilt.

Die Funktionsweise der Vorrichtung 50 zur Einstellung des Innendrucks in dem Abdichthohlkörper 52 soll im Folgenden anhand der Fig. 3 noch etwas erläutert werden. Es versteht sich, dass die folgenden Ausführungen auch für die Ausführungsformen gemäß Fig. 1 und Fig. 2 gelten.

Der Abdichthohlkörper 52 ist an einem distalen Ende 54 eines Endotrachealtubus 56 angeordnet. Er hat die Form einer um den Endotracheatubus 56 herum angeordneten Manschette, die einen Außenumfang des Endotrachealtubus 56 vollständig umgibt. Das distale Ende 54 des Endotrachealtubus 56 ist über die Rachenhöhle in die Luftröhre (Trachea) 58 eines zu beatmenden Patienten eingeführt. Damit liegt in dem in Fig. 3 gezeigten Zustand des Endotrachealtubus 56 ein Außenumfang des Abdichthohlkörpers 52 einer Innenwand der Trachea 58 gegenüber. Über den einen Innenraum des Abdichthohlkörpers 52 durchsetzenden Endotrachealtubus 58 kann Beatmungsluft der Lunge 60 des Patienten zugeführt werden bzw. ausgeatmete Luft aus der Lunge 60 abgeführt werden. Hierzu ist ein nicht im einzelnen dargestelltes proximales Ende des Endotrachealtubus 56 in bekannter Weise mit einer Beatmungsvorrichtung, beispielsweise der Beatmungsvorrichtung 10 aus Figur 1 oder 2 verbunden. Der Abdichthohlkörper 52 ist über die Druckluftleitung 62, ein Absperrventil 64 und den Druckluftschlauch 66 mit der Bedien/Steuereinheit 70 der Vorrichtung 50 zum Einstellen des Innendrucks in dem Abdichthohlkörper 52 verbunden. Die Bedien/Steuereinheit 70 ist einer Vorrichtung zur Erzeugung von Druckluft zugeordnet, um den Druckluftschlauch 66 mit einem vorbestimmten Druck, der mittels der Bedien/Steuereinheit 70 einstellbar ist, zu beaufschlagen. Die Druckerzeugungsvorrichtung kann in die Bedien/Steuereinheit 70 integriert sein oder aber mit der Bedien/Steuereinheit 70 in Verbindung stehen. Bei geöffnetem Absperrventil 64 wird die Leitung 62 und der von dem Abdichthohlkörper 52 umschlossene Innenraum ebenfalls mit diesem Druck beaufschlagt. Der Abdichthohlkörper 52 ist aus einem genügend elastischen Material gefertigt, so dass sich dar Abdichthohlkörper 52 bei Beaufschlagung mit Druck ausdehnt und an die Innenwände der Trachea 58 anlegt. Das distale Ende 54 des Endotrachealtubus 56 ist jedenfalls in dem Bereich, in dem es den Abdichthohlkörper 52 durchsetzt, aus einem ausreichend steifen Material hergestellt, so dass sich sein Querschnitt gar nicht, oder jedenfalls nur wenig, verringert, wenn der Innenraum des Abdichthohlkörpers 52 mit Druck beaufschlagt wird. Damit lässt sich durch Beaufschlagung des Abdichthohlkörpers 52 mit genügend großem Druck eine Abdichtung des Abdichthohlkörpers 52 gegenüber den Innewänden der Trachea 58 erzielen. Auf diese Weise lässt sich ein zwischen Lunge 60 und Abdichthohlkörper 52 liegender Bereich der Trachea 58 gegenüber der Umgebung, insbesondere gegenüber einem außerhalb liegenden Bereich der Trachea 58 (genauer ausgedrückt, gegenüber einem zwischen Abdichthohlkörper 52 und Rachenhöhle liegenden Bereich der Trachea 58) abdichten. Damit lässt sich insbesondere verhindern, dass Luft oder Sekrete aus dem Rachenbereich in den inneren Bereich der Trachea 58 und in die Lunge 60 gelangen können, während der Endotrachealtubus 56 in die Trachea 58 eingeführt ist.

Zur Überwachung der Dichtigkeit des Sitzes des Abdichthohlkörpers 52 gegenüber der Innenwand der Trachea 58 ist ein CO2-Sensor 68 in einem nicht durch den Abdichthohlkörper 52 abgedichteten Bereich der Trachea 58 angeordnet. In Fig. 3 sieht man, dass sich der CO2-Sensor 68 in einem Bereich zwischen dem Abdichthohlkörper 58 und der Rachenhöhle des Patienten befindet. Ein Anstieg des von dem CO2-Sensor 68 erfassten CO2-Signals weist darauf hin, dass vom Patienten ausgeatmete Luft zwischen dem Abdichthohlkörper 52 und der Innenwand der Trachea 58 entweichen kann und deutet damit auf einen undichten Sitz hin.

Fig. 4 zeigt ein Blockdiagramm, welches wesentliche Funktionseinheiten in einer zur automatisierten Bestimmung von Beatmungsparametern ausgebildeten Steuereinheit 100 einer Beatmungsvorrichtung 10 gemäß Fig. 1 oder 2 darstellt, gemäß einem ersten Beatmungsmodus "ASV" 102. Bei diesem Beatmungsmodus stellt die Steuereinheit 100 bestimmte Beatmungsparameter wie beispielsweise die Beatmungsfrequenz 110, das Tidalvolumen 112, die Dauer eines Inspirationsyklus 114 sowie den Beatmungsmodus 116 automatisch ein, während andere Beatmungsparameter wie beispielsweise das Minutenvolumen (hierunter versteht man das Produkt aus Tidalvolumen und Beatmungsfrequenz) 120 oder der positive endexpiratorische Druck PEEP 122 manuell eingestellt bzw. vorgegeben werden. Außerdem werden manuell noch bestimmte physiologische Parameter des zu beatmenden Patienten vorgegeben, beispielsweise dessen Größe 118. Die eigentliche Beatmung 104 wird dabei auf Grundlage der mittels der ASV Prozeduren ermittelten Beatmungsparameter 110 bis 116 sowie einigen der manuell vorgegebenen Parameter 120, 122 durchgeführt, wobei während der Beatmung noch bestimmte physiologische Parameter gemessen werden (in Fig. 2 ist beispielhaft der Atemgasfluss 124 genannt), welche dann ebenfalls als Grundlage für die automatische Festlegung der anderen Beatmungsparameter 110 bis 116 mittels ASV 104 dienen.

Die Steuereinheit 100 enthält ebenfalls eine Routine 160 zur Einstellung des Innendrucks in dem Abdichthohlkörper 52. Diese Routine 160 erhält als Eingangssignale den aktuell erfassten Innendruck 162 in dem Abdichthohlkörper 52 sowie ein Signal 164, das die von dem CO2-Sensor 68 erfasste CO2-Konzentration in einem nicht von dem Abdichthohlkörper 52 abgedichteten Volumen der Trachea 58 anzeigt. Anhand dieser Signale bestimmt die Routine 160 einen Sollwert für den Innendruck in dem Abdichthohlkörper 52 und gibt diesen Wert als Ausgangssignal 166 aus.

Figur 5 zeigt ein weiteres Blockdiagramm, welches wesentliche Funktionseinheiten in einer zur automatisierten Bestimmung von Beatmungsparametern ausgebildeten Steuereinheit 100 einer Beatmungsvorrichtung gemäß Fig. 1 oder 2 darstellt, gemäß einem noch weiter automatisierten Beatmungsmodus "INTELLIVENT-ASM" 140. Der Beatmungsmodus 140 gemäß Figur 5 arbeitet weitgehend automatisiert und umfasst neben bereits mit Bezug zu Figur 4 erläuterten Prozeduren des ASV-Modus 102 noch Prozeduren zur Steuerung von Ventilation 106 und Prozeduren zur Steuerung von Oxygenierung 108. Bei dem Beatmungsmodus 140 stellt die Steuereinheit 100 praktisch alle relevanten Beatmungsparameter weitgehend automatisiert ein, wie beispielsweise das Minutenvolumen 120, das sich wiederum aufteilt in Beatmungsfrequenz 110 und das Tidalvolumen 112, die Dauer eines Inspirationsyklus 114, den Beatmungsmodus 116, sowie mittels der Oxygenierungsprozeduren 106 auch den PEEP 120 und den Sauerstoffgehalt des Beatmungsgases 130. Als Eingangsgröße für die Berechnung dieser Beatmungsparameter dienen im Wesentlichen während der Beatmung 104 erfasste physiologische Parameter wie Atemgasfluss 124, CO2-Gehalt der ausgeatmeten Luft 134 oder Sauerstoffgehalt im arteriellen Blut des Patienten. Diese Parameter werden den Routinen 104, 106, 108 für die automatische Festlegung der anderen Beatmungsparameter 110, 112, 114, bis 116, 120 ,122, 130 als Eingangsgrößen zugeführt. Außerdem werden manuell noch bestimmte physiologische Parameter des zu beatmenden Patienten vorgegeben, beispielsweise dessen Größe 118 oder bestimmte Erkrankungen 126, oder bestimmte Strategien zur Entwöhnung des Patienten von der Beatmung 128. Damit läuft die Beatmung in diesem Beatmungsmodus an sich völlig automatisiert ab und erfordert an sich keine manuellen Eingriffe seitens einer Bedienperson.

Auch die Steuereinheit 100 gemäß Fig. 5 enthält die oben beschriebenen Routine 160 zur Einstellung des Innendrucks in dem Abdichthohlkörper 52, auf deren Beschreibung hiermit verwiesen wird.

Die vorliegende Erfindung gestattet es nun, teilweise oder sogar weitgehend automatisiert ablaufende Beatmungsprozeduren wie die Routine 160 zur Einstellung des Innendrucks in dem Abdichthohlkörper 52, aber auch ASV 102 oder INTELLIVENT-ASV 140 lediglich als Hilfsmittel zur Unterstützung von Bedienpersonen bei der manuellen Einstellung von Beatmungsparametern zur mechanischen Beatmung, insbesondere des Innendrucks in dem Abdichthohlkörper 52 einzusetzen. Die Möglichkeit, die Beatmung völlig ohne manuelle Eingriffe durchzuführen, wird also bewusst zurückgestellt und stattdessen wird einer Bedienperson eine möglichst leicht erfassbare und nicht störende Unterstützung bei der korrekten Einstellung von Beatmungsparametern "per Hand" angeboten. Aus diesem Grund werden die jeweils durch die automatischen Beatmungsprozeduren Routine 160 zur Einstellung des Innendrucks in dem Abdichthohlkörper 52, ASV 102 bzw. INTELLIVENT-ASV 140 ermittelten Werte der Beatmungsparameter 166, 110 - 116 bzw. 110, 112, 114, bis 116, 120 ,122, 130 nicht automatisch der eigentlichen Beatmung 104 zugrunde gelegt, sondern es wird zuvor die Möglichkeit angeboten, diese Beatmungsparameter manuell mittels Manipulation des Hauptbedienknopfs 22 bzw. des Manipulationsglieds 72 einzustellen. Dies ist in den Figuren 3 und 4 dadurch angedeutet, dass die Verbindung zwischen den ermittelten Beatmungsparametern 166, 110 - 116 bzw. 166, 110, 112, 114, bis 116, 120 ,122, 130 und der Beatmung 104 jeweils an der mit 150 bezeichneten Stelle unterbrochen ist. Hier findet also eine Möglichkeit der manuellen Eingabe von Beatmungsparametern statt, bei der die automatisiert ermittelten Werte ggf. auch ganz anders eingestellt werden können. Man kann das so ausdrücken, dass die Beatmungsvorrichtung 10 bzw. die Vorrichtung 50 zur Einstellung des Innendrucks in dem Abdichthohlkörper 52 zwar über ganz oder weitgehend vollständig automatisierte Beatmungsmodi bzw. Einstellmodi verfügt und diese auch bereitstellt, allerdings nicht zur Automatisierung der eigentlich stattfindenden Beatmung bzw. Einstellung des Innendrucks, sondern lediglich im Hintergrund als eine Unterstützung für Bedienpersonen bei der manuellen Einstellung eines dieser Parameter, insbesondere bei der Einstellung des Innendrucks in dem Abdichthohlkörper 52. Diese Unterstützung soll durch eine haptische Rückkopplung erfolgen, welche auf ein zur manuellen Einstellung der Beatmungsparameter zu betätigendes Manipulationsglied 22 oder 72 urückwirkt.

Weil die im Hintergrund ablaufenden teilweise oder weitgehend automatischen Beatmungsmodi laufend bevorzugte Werte für die jeweils einzustellenden Beatmungsparameter, insbesondere den Innendruck in dem Abdichthohlkörper 52, liefern, kann diese haptische Rückkopplung in einer solchen Weise erfolgen, dass die jeweilige Bedienperson eine fühlbare Rückmeldung erhält, wenn sie einen Wert für einen der Beatmungsparameter, insbesondere den Innendruck in dem Abdichthohlkörper 52, einstellt, die mit dem von der im Hintergrund ablaufenden Routine eingestellten Wert übereinstimmt.

Die Haptik-Rückkopplung kann eine erste Haptik-Rückkopplung umfassen (siehe Fig. 6), die dazu ausgebildet ist, den Hauptbedienknopf 22 bzw. das Manipulationsglied 72 im Sinne einer ersten haptischen Rückkopplung derart anzusteuern, dass eine dem von der Steuereinheit bestimmten Sollwert des wenigstens einen Beatmungsparameters, insbesondere des Innendrucks in dem Abdichthohlkörper 52, entsprechende Stellung des Hauptbedienknopfes 22 bzw. des Manipulationsglieds für die Bedienperson wahrnehmbar hervorgehoben wird. Alternativ oder zusätzlich kann die erste Haptik-Rückkopplungseinheit dazu ausgebildet sein, den Hauptbedienknopf 22 bzw. das Manipulationsglied 72 derart anzusteuern, dass die dem von der Steuereinheit bestimmten Sollwert des wenigstens einen Beatmungsparameters, insbesondere der Einstellung des Innendrucks in dem Abdichthohlkörper 52, entsprechende Stellung des Hauptbedienknopfes 22 bzw. des Manipulationsglieds 72 für die Bedienperson fühlbar hervorgehoben wird.

Alternativ oder zusätzlich kann die Haptik-Rückkopplungseinheit noch eine zweite Haptik-Rückkopplungseinheit umfassen, die eine zweite haptische Rückmeldung liefert, die den Hauptbedienknopf 22 bzw. das Manipulationsglied 72 abhängig von einem Ist-Zustand des wenigstens einen Beatmungsparameters, insbesondere des Innendrucks in dem Abdichthohlkörper 52, beaufschlagt. Die zweite haptische Rückkopplung ergibt damit ein fühlbares Signal entsprechend der Verstellung des Hauptbedienknopfs 22 bzw. des Manipulationsglieds 72 im Zuge einer Manipulation, das die damit einhergehende Veränderung des einzustellenden Beatmungsparameters erfühlbar werden lässt.

Insgesamt ergibt sich dann eine haptische Rückkopplung, die einer Überlagerung der ersten haptischen Rückkopplung mit der zweiten haptischen Rückkopplung entspricht, wie dies in Fig. 7 gezeigt ist.

Es versteht sich, dass die vorangehende Beschreibung auch hinsichtlich der in Fig. 3 gezeigten Ausführungsform gilt, wobei in diesem Fall die Steuereinheit 70 dazu ausgebildet ist, die Routine 160 zur Einstellung des Innendrucks in dem Abdichthohlkörper 52 abzuarbeiten unabhängig davon, ob die Steuereinheit 70 mit einer Beatmungsvorrichtung gekoppelt ist und unabhängig von einem in einer Beatmungsvorrichtung eingestellten manuellen oder automatischen Beatmungsmodus.

Fig. 6 zeigt in schematischer Ansicht den Verlauf eines Widerstands W gegenüber einer Verstellbewegung um einen Winkel m des Hauptbedienknopfs 22 bzw. des Manipulationsglieds 72 im Zuge einer manuellen Einstellung des Innendrucks in dem Abdichthohlkörper 52 gemäß einer Ausführungsform, bei der nur eine erste haptische Rückmeldung vorhanden ist. Die erste haptische Rückmeldung erzeugt ein fühlbares Klick-Gefühl oder Einrast-Gefühl, wenn die Stellung des Hauptbedienknopfs 22 bzw. des Manipulationsglieds 72 im Zuge einer Manipulationsbewegung zur Einstellung eines neuen Werts für den innendruck in dem Abdichthohlkörper 52 eine Stellung S erreicht bzw. überschreitet, die dem von der im Hintergrund ablaufenden Routine 160 automatisch bestimmten Wert entspricht. Wird im Zuge einer Verstellbewegung, beispielsweise ausgehend von einer Anfangsstellung A des Hauptbedienknopfs 22 bzw. des Manipulationsglieds 72 aus in Richtung zu einer Endstellung E des Hauptbedienknopfs 22 bzw. des Manipulationsglieds 72 hin, diese dieser Stelle S erreicht, verringert sich nämlich ein einer weiteren Verstellbewegung entgegengesetzter Widerstand W (beispielsweise in Form eines von der Haptik-Rückkopplungseinheit ausgeübten der Verstellbewegung entgegen wirkenden Gegen-Drehmoments) abrupt. Sobald diese Stelle S überschritten ist, erhöht sich der Widerstand wieder genauso abrupt, was dem Bediener das Gefühl eines Einrastens oder Klickens gibt, wenn der Hauptbedienknopf 22 bzw. das Manipulationsglied 72 die Stellung S erreicht bzw. überschreitet, die dem von der im Hintergrund ablaufenden Routine 160 automatisch bestimmten Wert entspricht.

Zusätzlich kann noch vorgesehen sein, dass der Widerstand W dann abrupt auf Null oder nahezu Null zurückgeht, wenn eine Endstellung E erreicht ist, die einem maximal höchstens zulässigen oder einem minimal mindestens erforderlichen Wert des einzustellenden Parameters entspricht. Damit stellt sich beim Bediener ein "Leerlaufgefühl" ein, wenn die Endstellung E erreicht ist, so dass der Bediener ohne Weiteres merkt, dass eine weitere Verdrehung des Hauptbedienknopfs 22 bzw. des Manipulationsglieds 72 in derselben Richtung keine weitere Veränderung des eingestellten Werts des Innendrucks in dem Abdichthohlkörper 52 mehr bringen wird.

Die Darstellung in Fig. 7 entspricht der in Fig. 6. Gezeigt ist also der Verlauf des Widerstandes W gegenüber einer Weiterbewegung in derselben Richtung als Funktion einer Verlagerung des Hauptbedienkopfs 22 bzw. des Manipulationsglieds 72 um einen Winkel m zwischen einer Anfangsstellung A und einer maximal zulässigen Endstellung E. Bei der in Fig. 7 gezeigten Ausführungsform ist zusätzlich zu der der mit Bezug zu Fig.6 bereits erläuterten ersten haptischen Rücckopplung bei Erreichen der Sollstellung S zusätzlich noch eine zweite haptische Rückkopplung der ersten haptischen Rückkopplung überlagert. Die zweite haptische Rückkopplung zeigt eine Veränderung des Innendrucks in dem Abdichthohlkörper 52 mit weiter zunehmender Verstellung des Hauptbedienknopfs 22 bzw. des Manipulationsglieds 72 an. Damit erhält die Bedienperson bereits im Zuge einer Verstellung des Hauptbedienknopfs 22 bzw. des Manipulationsglieds 72 eine fühlbare Rückmeldung, in welcher Weise sich der Innendruck in dem Abdichthohlkörper 52 verändern wird, wenn die Neueinstellung wirksam geworden ist. Gerade erfahrene Bedienpersonen sind in der Lage, bereits anhand einer solchen fühlbaren Rückmeldung eine gute Abschätzung eines geeigneten Werts für den Parameter vorzunehmen. Ein Vorteil ist, dass die hier vorgeschlagene Vorgehensweise es der Bedienperson ermöglicht, den von ihr erfühlten oder abgeschätzten Wert für den Innendruck in dem Abdichthohlkörper 52 mit dem von der automatisierten Routine 160 vorgeschlagenen Wert zu vergleichen. Außerdem besteht die Möglichkeit, den neuen Wert zunächst nur einzustellen durch Verdrehen des Hauptbedienknopfes 22 bzw. Manipulationsglieds 72 bis zur gewünschten Stellung und erst nach erfolgter Einstellung in der Beatmung wirksam werden zu lassen (etwa durch Drücken des Hauptbedienknopfs 22 bzw. Manipulationsglieds 72). Man kann also nochmals nachjustieren, wenn beispielsweise einmal eine große Diskrepanz zwischen dem von der Bedienperson erfühlten idealen Wert und dem von der automatisierten Routine 160 vorgeschlagenen Wert auftritt.

Bei der Ausführungsform gemäß Fig. 7 ist vorgesehen, dass der Widerstand W abrupt auf einen sehr hohen Wert ansteigt, wenn eine Endstellung E erreicht ist, die einem maximal höchstens zulässigen oder einem minimal mindestens erforderlichen Wert des Innendrucks in dem Abdichthohlkörper 52 entspricht. Damit stellt sich beim Bediener das Gefühl eines Anschlags oder einer Endbegrenzung, gegen die der Hauptbedienknopf 22 bzw. das Manipulationsglied 72 nicht mehr weiter verdreht werden kann. Auch auf diese Weise lässt sich erreichen, dass die Bedienperson den Hauptbedienknopf 22 bzw. das Manipulationsglied nicht über einer Endstellung E hinaus verdrehen kann. Selbstverständlich kann die in Fig. 6 gezeigte Variante bei Erreichen der Endstellung E in der Ausführungsform gemäß Fig. 7 Anwendung finden, falls gewünscht. Umgekehrt kann selbstverständlich die in Fig. 7 gezeigte Variante bei Erreichen der Endstellung E in der Ausführungsform gemäß Fig. 6 Anwendung finden, falls gewünscht.

## Patentansprüche

1. Vorrichtung (10; 50) zur Einstellung eines Innendrucks in einem Abdichthohl - körper (52), umfassend:
einen Trachealtubus (56) mit einem distalen Abschnitt (54), der zur Abgabe von Beatmungsluft in eine Trachea (58) eines Patienten ausgebildet ist;
einen Abdichthohlkörper (52), der dem distalen Abschnitt des Trachealtubus (56) zur Abgabe von Beatmungsluft in eine Trachea (58) eines Patienten zuge - ordnet ist,
eine Bedienvorrichtung mit einem Manipulationsglied (22; 72), welches zur manuellen Einstellung des Innendrucks in dem Abdichthohlkörper (52) durch eine Bedienperson manuell betätigbar ist,
eine Steuereinheit (70; 100), dazu ausgebildet ist, auf Grundlage von erfassten Werten von Kenngrößen einen Sollwert für den Innendruck in dem Ab dichthohlkörper (52) automatisiert zu bestimmen;
wobei der Bedienvorrichtung eine erste Haptik-Rückkopplungseinheit zugeordnet ist, die dazu ausgebildet ist, eine auf Grundlage einer Abweichung zwischen dem durch manuelle Betätigung des Manipulationsglieds (22, 72) aktuell eingestellten Wert des Innendrucks in dem Ab dichthohlkörper (52) und dem von der Steuereinheit (70; 100) bestimmten Sollwert des Innendrucks in dem Abdichthohlkörper (52) bestimmte erste haptische Rückkopplung zu erzeugen.

2. Vorrichtung (10; 50) nach Anspruch 1, wobei der Abdichthohlkörper (52) zur Abdichtung eines Zwischenraums zwischen dem in die Trachea (58) eingeführten distalen Abschnitt (54) des Trachealtubus (56) und einer Innenwand der Trachea (58) ausgebildet ist.

3. Vorrichtung (10; 50) nach Anspruch 1 oder 2, wobei der Abdichthohlkörper (52) die Konfiguration einer um einen Umfang am distalen Abschnitt (54) des Trachealtubus (56) herum angeordneten Manschette hat.

4. Vorrichtung (10; 50) nach einem der Ansprüche 1 bis 3,wobei der Trachealtubus (56) als Endotrachealtubus zum Einführen in die Trachea (58) über die Rachenhöhle des Patienten oder als Tracheostomietubus zum Einführen in die Trachea (58) mittels Luftröhrenschnitt ausgebildet ist.

5. Vorrichtung (10; 50) nach einem der Ansprüche 1 bis 4, wobeidie Steuerein - heit (70) als portables Gerät ausgebildet ist und das Manipulationsglied (72) an der Steuereinheit (70) gelagert ist.

6. Vorrichtung (10; 50) nach Anspruch 5, wobei die erste Haptik-Rückkopp - lungseinheit dazu ausgebildet ist, das Manipulationsglied (22; 72) im Sinne der ersten haptischen Rückkopplung derart anzusteuern, dass eine dem von der Steuereinheit (70; 100) bestimmten Sollwert des Innendrucks in dem Abdichthohlkörper (52) entsprechende Stellung des Manipulationsglieds (22; 72) für die Bedienperson wahrnehmbar hervorgehoben wird; und/oder
wobei die Steuereinheit (70; 100) dazu ausgebildet ist, den Sollwert für den Innendruck in dem Abdichthohlkörper (52) fortlaufend, insbesondere synchronisiert mit jeweiligen Atemzyklen, automatisiert zu bestimmen; und/oder
wobei die erste Haptik-Rückkopplungseinheit dazu ausgebildet ist, das Manipulationsglied (22; 72) derart anzusteuern, dass die dem von der Steuereinheit (70; 100) bestimmten Sollwert des Innendrucks in dem Abdichthohlkörper (52) entsprechende Stellung des Manipulationsglieds (22; 72) für die Bedienperson fühlbar hervorgehoben wird; und/oder
wobei die erste Haptik-Rückkopplungseinheit das Manipulationsglied (22; 72) in einer solchen Weise beaufschlagt, dass das Manipulationsglied (22; 72) ohne manuelle Betätigung eine Stellung einnimmt - oder jedenfalls einzunehmen sucht -, die dem durch die Steuereinheit (70; 100) bestimmten Sollwert des Innendrucks in dem Abdichthohlkörper (52) entspricht; und/oder
wobei die erste Haptik-Rückkopplungseinheit das Manipulationsglied (70; 100) in einer solchen Weise beaufschlagt, dass das Manipulationsglied (70; 100) in eine Stellung zurückkehrt - oder jedenfalls in eine solche Stellung zurückzukehren sucht -, die dem durch die Steuereinheit (70; 100) bestimmten Sollwert des Innendrucks in dem Abdichthohlkörper (52) entspricht, wenn nach erfolgter manueller Betätigung das Manipulationsglied (22; 72) wieder freigegeben wird.

7. Vorrichtung (10; 50) nach Anspruch 5 oder 6, wobei die erste Haptik-Rücckopplungseinheit dazu ausgebildet ist, im Zuge eines manuellen Manipulationsvorgangs des Manipulationsglieds (22; 72) bei Überfahren einer Stellung des Manipulationsglieds (22; 72), die dem von der Steuereinheit (70; 100) bestimmten Sollwert des Innendrucks in dem Abdichthohlraum (52) entspricht, eine abrupte Änderung der ersten haptischen Rückkopplung zu erzeugen.

8. Vorrichtung (10; 50) nach einem der Ansprüche 1 bis 7, wobei der ersten Haptik-Rückkopplungseinheit wenigstens ein Sensor zugeordnet ist, der zur Erfassung der Stellung des Manipulationsglieds (22; 72), insbesondere in Bezug auf eine Referenzstellung, ausgebildet ist; und/oder
wobei die zweite Haptik-Rückkopplungseinheit das Manipulationsglied abhängig von einem Ist-Zustand des Innendrucks in dem Abdichthohlkörper (72) im Sinne einer zweiten haptischen Rückkopplung beaufschlagt.

9. Vorrichtung (10; 50) nach Anspruch 8, wobei die zweite Haptik-Rückkopplungseinheit das Manipulationsglied (22; 72) mit einer den Ist-Zustand des Innendrucks in dem Abdichthohlkörper (52) anzeigenden zweiten haptischen Rücckopplung beaufschlagt; und/oder
wobei der zweiten Haptik-Rückkopplungseinheit wenigstens ein Sensor zugeordnet ist, der zur Erfassung des Innendrucks in dem Abdichthohlkörper (52) ausgebildet ist.

10. Vorrichtung (10; 50) nach einem der Ansprüche 1 bis 9, ferner umfassend wenigstens einen Sensor (68) zur Erfassung der CO2 Konzentration in einem nicht von dem Abdichthohlkörper (52) abgedichteten Bereich der Trachea (58).

11. Vorrichtung (10; 50) nach einem der Ansprüche 1 bis 10, wobei das Manipulationsglied (22; 72) als Drehglied ausgebildet ist, oder ein als Drehglied ausgebildetes Einstellelement umfasst, oder
als Dreh-/Drück-Steller ausgebildet ist.

12. Beatmungsvorrichtung (10) zur maschinellen Beatmung von Patienten, umfassend eine Vorrichtung (50) nach einem der vorangehenden Ansprüche zur Einstellung eines Innendrucks in einem Abdichthohlkörper (52), der einem distalen Abschnitt (54) eines Trachealtubus (56) zur Abgabe von Beatmungsluft in eine Trachea (58) eines Patienten, zugeordnet ist; und
wobei das Manipulationsglied (22, 72) zur Einstellung von mehreren Beatmungsparametern ausgebildet ist.

13. Verfahren zum Bereitstellen einer unterstützenden Interaktion beim Einstellen eines Innendrucks in einem einem Trachealtubus (56) zugeordneten Abdichthohlkörper (52), bei welchem ein einer Bedienvorrichtung zugeordnetes Manipulationsglied (22; 72) zum Einstellen des Innendrucks in dem Abdichthohlkörper (52) durch eine Bedienperson manuell betätigt wird, und das Manipulationsglied (22; 72) in Reaktion auf die manuelle Betätigung des Manipulationsglieds (22; 72) zur Einstellung des Innendrucks in dem Abdichthohlkörper (52) im Sinne einer ersten haptischen Rückkopplung angesteuert wird,
wobei auf Grundlage von erfassten Werten von Kenngrößen (162, 164) ein Soll - wert (166) für den Innendruck in dem Abdichthohlkörper (52) durch eine Steuereinheit (70; 100) automatisiert bestimmt wird, und das Manipulationsglied (22; 72) während der manuellen Betätigung im Sinne der ersten haptischen Rück - kopplung derart angesteuert wird, dass eine einer Abweichung zwischen dem durch manuelle Betätigung des Manipulationsglieds aktuell eingestellten Wert des Innendrucks in dem Abdichthohlkörper (52) und dem von der Steuereinheit (70; 100) bestimmten Sollwert (166) des Innendrucks in dem Abdichthohlkörper (52) entsprechende Stellung des Manipulationsglieds (22; 72) für die Bedienperson wahrnehmbar, insbesondere fühlbar, hervorgehoben wird.

14. Computerprogrammprodukt, welches Programmanweisungen enthält, bei de - ren Ausführung auf einer Vorrichtung nach einem der Ansprüche 1 bis 12 ein Ver - fahren nach Anspruch 13 ausgeführt wird.

## Claims

1. A device (10; 50) for adjusting an internal pressure in a hollow sealing body (52), comprising:
a tracheal tube (56) having a distal section (54) configured to deliver ventila - tion air into a trachea (58) of a patient;
a hollow sealing body (52) which is associated with the distal section of the tracheal tube (56) for delivering ventilation air into a trachea (58) of a pa - tient,
an operating device having a manipulation member (22; 72) which can be operated manually by an operator for manual adjustment of the internal pressure in the hollow sealing body (52),
a control unit (70; 100) which is designed to determine in automated man - ner a target value for the internal pressure in the hollow sealing body (52) on the basis of detected values of parameters,
wherein the operating device has a first haptic feedback unit associated therewith, which is designed to generate a first haptic feedback that is de - termined on the basis of a deviation between the value of the internal pres - sure in the hollow sealing body currently set by manual operation of the ma - nipulation member (22, 72) and the target value of the internal pressure in the hollow sealing body (52) determined by the control unit (70; 100).

2. The device (10; 50) according to claim 1,
wherein the hollow sealing body (52) is configured to seal a space between the distal section (54) of the tracheal tube (56) inserted into the trachea (58) and an inner wall of the trachea (58).

3. The device (10; 50) according to claim 1 or 2,
wherein the hollow sealing body (52) has the configuration of a cuff dis - posed about a circumference at the distal section (54) of the tracheal tube (56).

4. The device (10; 50) according to any of claims 1 to 3,
wherein the tracheal tube (56) is designed as an endotracheal tube for insertion into the trachea (58) via the pharynx of the patient or as a tracheostomy tube for insertion into the trachea (58) by means of a tracheotomy.

5. The device (10; 50) according to any of claims 1 to 4,
wherein the control unit (70) is designed as a portable device and the manipulation member (72) is supported on the control unit (70).

6. The device (10; 50) according to claim 5,
wherein the first haptic feedback unit is designed to control the manipulation member (22; 72) in the sense of the first haptic feedback in such a way that a position of the manipulation member (22; 72) corresponding to the target value of the internal pressure in the hollow sealing body (52) determined by the control unit (70; 100) is emphasized perceptibly for the operator; and/or wherein the control unit (70; 100) is designed to determine in automated manner the target value for the internal pressure in the hollow sealing body (52) continuously, in particular synchronized with the respective breathing cycles; and/or
wherein the first haptic feedback unit is designed to control the manipulation member (22; 72) in such a way that the position of the manipulation member (22; 72) corresponding to the target value of the internal pressure in the hollow sealing body (52) determined by the control unit (70; 100) is tangibly emphasized for the operator; and/or
wherein the first haptic feedback unit acts on the manipulation member (22; 72) in such a way that the manipulation member (22; 72) without manual operation assumes a position - or at least seeks to assume such a positionwhich corresponds to the target value of the internal pressure in the hollow sealing body (52) determined by the control unit (70; 100); and/or
wherein the first haptic feedback unit acts on the manipulation member (70; 100) in such a way that the manipulation member (70; 100) returns to a position - or at least seeks to return to such a position - which corresponds to the target value of the internal pressure in the hollow sealing body (52) determined by the control unit (70; 100) when the manipulation member (22; 72) is released again after manual operation thereof has taken place.

7. The device (10; 50) according to claim 5 or 6,
wherein the first haptic feedback unit, in the course of a manual manipula - tion process of the manipulation member (22; 72) when a position of the manipulation member (22; 72) is passed corresponding to the target value of the internal pressure in the hollow sealing body (52) determined by the control unit (70; 100), is designed to generate an abrupt change in the first haptic feedback.

8. The device (10; 50) according to any of claims 1 to 7,
wherein the first haptic feedback unit has at least one sensor associated therewith which is designed to detect the position of the manipulation mem - ber (22; 72), in particular in relation to a reference position; and/or
wherein the second haptic feedback unit acts on the manipulation member depending on an actual state of the internal pressure in the hollow sealing body (72) in the sense of a second haptic feedback.

9. The device (10; 50) according to claim 8,
wherein the second haptic feedback unit applies a second haptic feedback to the manipulation member (22; 72) indicative of the actual state of the in - ternal pressure in the hollow sealing body (52); and/or
wherein the second haptic feedback unit has at least one sensor associated therewith which is designed to detect the internal pressure in the hollow sealing body (52).

10. The device (10; 50) according to any of claims 1 to 9,
further comprising at least one sensor (68) for detecting the CO2 concentra - tion in a portion of the trachea (58) which is not sealed by the hollow sealing body (52).

11. The device (10; 50) according to any of claims 1 to 10,
wherein the manipulation member (22; 72) is designed as a rotating mem - ber or comprises an adjustment element designed as a rotating member, or is designed as a rotary/push actuator.

12. A ventilation device (10) for mechanical ventilation of patients, comprising a device (50) according to any of the preceding claims for adjusting an in - ternal pressure in a hollow sealing body (52) associated with a distal section (54) of a tracheal tube (56) for delivering ventilation air into a trachea (58) of a patient;
wherein the manipulation member (22, 72) is designed for adjusting a plural - ity of ventilation parameters.

13. A method for providing a supporting interaction in adjusting an internal pres - sure in a hollow sealing body (52) associated with a tracheal tube (56), in which a manipulation member (22; 72) associated with an operating device is manually operated by an operator for adjusting the internal pressure in the hollow sealing body (52), and the manipulation member (22; 72) is con - trolled in response to the manual operation of the manipulation member (22; 72) for adjusting the internal pressure in the hollow sealing body (52) in the sense of a first haptic feedback,
wherein a target value (166) for the internal pressure in the hollow sealing body (52) is determined in automated manner by a control unit (70; 100) on the basis of detected values of parameters (162, 164), and the manipulation member (22; 72) during manual operation in the sense of the first haptic feedback is controlled in such a way that a position of the manipulation member (22; 72) corresponding to a deviation between the value of the in - ternal pressure in the hollow sealing body (52) which is currently set by manual operation of the manipulation member and the target value (166) of the internal pressure in the hollow sealing body (52) determined by the con - trol unit (70; 100) is emphasized perceptibly, in particular tangibly, for the operator.

14. A computer program product containing program instructions which, when executed on a device according to any of claims 1 to 12, execute a method according to claim 13.

## Revendications

1. Dispositif (10 ; 50) de réglage d'une pression interne dans un corps creux d'étanchéité (52) comprenant :
une sonde trachéale (56) avec une partie distale (54) réalisée de manière à délivrer de l'air respirable dans la trachée (58) d'un patient ;
un corps creux d'étanchéité (52) affecté à la partie distale de la sonde trachéale (56) pour délivrer de l'air respirable dans la trachée (58) d'un patient ;
un dispositif de commande avec un élément de manipulation (22 ; 72) qui peut être actionné manuellement par un opérateur en vue d'un réglage manuel de la pression interne dans le corps creux d'étanchéité (52) ;
une unité de contrôle (70 ; 100) réalisée de manière à déterminer automatiquement une valeur de pression interne de consigne dans le corps creux d'étanchéité (52) sur la base de valeurs de paramètres saisies ;
sachant qu'une première unité de rétroaction haptique est affectée au dispositif de commande, unité qui est réalisée de manière à générer une certaine première rétroaction haptique sur la base d'un écart entre la valeur de la pression interne dans le corps creux d'étanchéité (52) actuellement réglée par l'actionnement manuel de l'élément de manipulation (22, 72) et la valeur de consigne de la pression interne dans le corps creux d'étanchéité (52) déterminée par l'unité de contrôle (70 ; 100).

2. Dispositif (10 ; 50) selon la revendication 1, dans lequel le corps creux d'étanchéité (52) est réalisé de manière à étanchéifier un espace situé entre la partie distale (54) de la sonde trachéale (56) introduite dans la trachée (58) et une paroi interne de la trachée (58).

3. Dispositif (10 ; 50) selon la revendication 1 ou 2, dans lequel le corps creux d'étanchéité (52) a la configuration d'un manchon disposé autour d'une circonférence de la partie distale (54) de la sonde trachéale (56).

4. Dispositif (10 ; 50) selon l'une quelconque des revendications 1 à 3, dans lequel la sonde trachéale (56) est réalisée en tant que sonde endotrachéale destinée à être introduite dans la trachée (58) par le pharynx du patient ou en tant que sonde de trachéotomie à introduire dans la trachée (58) au moyen d'une trachéotomie.

5. Dispositif (10 ; 50) selon l'une quelconque des revendications 1 à 4, dans lequel l'unité de contrôle (70) est réalisée en tant qu'appareil portatif et l'élément de manipulation (72) est monté sur l'unité de contrôle (70).

6. Dispositif (10 ; 50) selon la revendication 5, dans lequel la première unité de rétroaction haptique est réalisée de manière à commander l'élément de manipulation (22 ; 72), s'agissant de la première rétroaction haptique, de telle sorte qu'une position de l'élément de manipulation (22 ; 72) correspondant à la valeur de consigne de la pression interne dans le corps creux d'étanchéité (52) déterminée par l'unité de contrôle (70 ; 100) soit mise en évidence de manière perceptible pour l'opérateur ; et/ou
dans lequel l'unité de contrôle (70 ; 100) est réalisée de manière à déterminer automatiquement la valeur de consigne de la pression interne dans le corps creux d'étanchéité (52) en continu, en particulier de manière synchronisée avec les cycles respiratoires respectifs ; et/ou
dans lequel la première unité de rétroaction haptique est réalisée de manière à commander l'élément de manipulation (22 ; 72) de telle sorte que la position de l'élément de manipulation (22 ; 72) correspondant à la valeur de consigne de la pression interne dans le corps creux d'étanchéité (52) déterminée par l'unité de contrôle (70 ; 100) soit mise en évidence de manière palpable pour l'opérateur ; et/ou
dans lequel la première unité de rétroaction haptique sollicite l'élément de manipulation (22 ; 72) de telle sorte que l'élément de manipulation (22 ; 72) prend - ou du moins cherche à prendre - une position sans actionnement manuel, qui correspond à la valeur de consigne de la pression interne dans le corps creux d'étanchéité (52) déterminée par l'unité de contrôle (70 ; 100) ; et/ou
dans lequel la première unité de rétroaction haptique sollicite l'élément de manipulation (22 ; 72) de telle sorte que l'élément de manipulation (22 ; 72) revient dans une position - ou du moins cherche à revenir dans une telle position - qui correspond à la valeur de consigne de la pression interne dans le corps creux d'étanchéité (52) déterminée par l'unité de contrôle (70 ; 100), lorsque, une fois l'actionnement manuel effectué, l'élément de manipulation (22 ; 72) est à nouveau libéré.

7. Dispositif (10 ; 50) selon la revendication 5 ou 6, dans lequel la première unité de rétroaction haptique est réalisée de manière à générer, au cours d'une opération de manipulation manuelle de l'élément de manipulation (22 ; 72), un changement brusque de la première rétroaction haptique en cas de dépassement d'une position de l'élément de manipulation (22 ; 72) qui correspond à la valeur de consigne de la pression interne dans le corps creux d'étanchéité (52) déterminée par l'unité de contrôle (70 ; 100).

8. Dispositif (10 ; 50) selon l'une quelconque des revendications 1 à 7, dans lequel au moins un capteur est affecté à la première unité de rétroaction haptique, capteur réalisé de manière à saisir la position de l'élément de manipulation (22 ; 72), en particulier par rapport à une position de référence ; et/ou
dans lequel la deuxième unité de rétroaction haptique sollicite l'élément de manipulation en fonction d'un état réel de la pression interne dans le corps creux d'étanchéité (52) aux fins d'une deuxième rétroaction haptique.

9. Dispositif (10 ; 50) selon la revendication 8, dans lequel la deuxième unité de rétroaction haptique sollicite l'élément de manipulation (22 ; 72) avec une deuxième rétroaction haptique affichant l'état réel de la pression interne dans le corps creux d'étanchéité (52) ; et/ou
dans lequel au moins un capteur est affecté à la deuxième unité de rétroaction haptique, capteur réalisé de manière à saisir la pression interne dans le corps creux d'étanchéité (52).

10. Dispositif (10 ; 50) selon l'une quelconque des revendications 1 à 9, comprenant en outre au moins un capteur (68) permettant de saisir la concentration de CO₂ dans une zone de la trachée (58) qui n'est pas étanchéifiée par le corps creux d'étanchéité (52).

11. Dispositif (10 ; 50) selon l'une quelconque des revendications 1 à 10, dans lequel l'élément de manipulation (22 ; 72) est réalisé en tant qu'élément rotatif, ou bien qu'il comprend un élément de réglage réalisé en tant qu'élément rotatif, ou bien qu'il est réalisé en tant qu'actionneur rotatif/poussoir.

12. Dispositif respiratoire (10) pour la ventilation mécanique de patients, comprenant un dispositif (50) selon l'une quelconque des revendications précédentes pour le réglage d'une pression interne dans un corps creux d'étanchéité (52), qui est affecté à une partie distale (54) d'une sonde trachéale (56) destinée à délivrer de l'air respirable dans la trachée (58) d'un patient ; et
dans lequel l'élément de manipulation (22 ; 72) est réalisé en vue du réglage de plusieurs paramètres respiratoires.

13. Procédé de mise à disposition d'une interaction de soutien lors du réglage d'une pression interne dans un corps creux d'étanchéité (52) affecté à une sonde trachéale (56), dans lequel un élément de manipulation (22 ; 72) affecté à un dispositif de commande est actionné manuellement par un opérateur en vue de régler la pression interne dans le corps creux d'étanchéité (52), et où l'élément de manipulation (22 ; 72), en réaction à l'actionnement manuel de l'élément de manipulation (22 ; 72), est commandé de manière à régler la pression interne dans le corps creux d'étanchéité (52) aux fins d'une première rétroaction haptique,
sachant que, sur la base de valeurs de paramètres saisies (162, 164), une valeur de consigne (166) de la pression interne dans le corps creux d'étanchéité (52) est déterminée automatiquement par une unité de contrôle (70 ; 100), et que l'élément de manipulation (22 ; 72), pendant l'actionnement manuel en vue de la première rétroaction haptique, est commandé de telle sorte qu'une position de l'élément de manipulation (22 ; 72) correspondant à un écart entre la valeur actuelle de la pression interne dans le corps creux d'étanchéité (52) réglée par actionnement manuel de l'élément de manipulation et la valeur de consigne (166) de la pression interne dans le corps creux d'étanchéité (52) déterminée par l'unité de contrôle (70 ; 100) est mise en évidence de manière perceptible, en particulier palpable, par l'opérateur.

14. Produit de programme informatique contenant des instructions dont l'exécution sur un dispositif selon l'une quelconque des revendications 1 à 12 entraîne l'exécution d'un procédé selon la revendication 13.
